# EUROPEAN PATENT SPECIFICATION

(11) **EP 3 094 973 B1**
(45) Date of publication and mention of the grant of the patent: **29.07.2020**
(21) Application number: 13802086.2
(22) Date of filing: 07.11.2013
(51) Int. Cl.: G01N 33/50

(54) **BIOMARKERS**
BIOMARKER
BIOMARQUEURS

(43) Date of publication of application: 23.11.2016
(73) Proprietor: Diagnodus Limited, Babraham, Cambridgeshire CB22 3AT (GB)
(72) Inventor: LOKTIONOV, Alexandre, Cambridge Cambridgeshire CB22 5EA (GB); BANDALETOVA, Tatiana, Cambridge Cambridgeshire CB22 5EA (GB)
(74) Representative: Williams, Andrea
(86) International application number: PCT/GB2013/052935
(87) International publication number: WO 2015/067913

(56) References cited:
- WO-A1-94/21662
- WO-A1-2012/150453
- WO-A2-2009/120877
- US-A- 5 928 883
- US-A1- 2010 255 513
- KUNA A T: "Serological markers of inflammatory bowel disease", BIOCHEMIA MEDICA 2013 BIOCHEMIA MEDICA, EDITORIAL OFFICE HRV, vol. 23, no. 1, January 2013 (2013-01), pages 28-42, XP002720419, ISSN: 1330-0962
- DAINESE RAFFAELLA ET AL: "Role of serological markers of activated eosinophils in inflammatory bowel diseases.", April 2012 (2012-04), EUROPEAN JOURNAL OF GASTROENTEROLOGY & HEPATOLOGY APR 2012, VOL. 24, NR. 4, PAGE(S) 393 - 397, XP002720420, ISSN: 1473-5687 abstract
- Seung-Woo Shin ET AL: "Elevation of Eosinophil-Derived Neurotoxin in Plasma of the Subjects with Aspirin-Exacerbated Respiratory Disease: A Possible Peripheral Blood Protein Biomarker", PLoS ONE, vol. 8, no. 6, 21 June 2013 (2013-06-21), page e66644, XP55413125, DOI: 10.1371/journal.pone.0066644
- Michael Wagner: "Fecal markers of inflammation used as surrogate markers for treatment outcome in relapsing inflammatory bowel disease", World Journal of Gastroenterology, vol. 14, no. 36, 1 January 2008 (2008-01-01), page 5584, XP055413309, CN ISSN: 1007-9327, DOI: 10.3748/wjg.14.5584
- Seung-Woo Shin ET AL: "Elevation of Eosinophil-Derived Neurotoxin in Plasma of the Subjects with Aspirin-Exacerbated Respiratory Disease: A Possible Peripheral Blood Protein Biomarker", PLoS ONE, vol. 8, no. 6, 21 June 2013 (2013-06-21), page e66644, XP055413125, DOI: 10.1371/journal.pone.0066644
- ALEXANDRE LOKTIONOV ET AL: "Inflammatory bowel disease detection and monitoring by measuring biomarkers in non-invasively collected colorectal mucus : Inflammation biomarkers in colorectal mucus", JOURNAL OF GASTROENTEROLOGY AND HEPATOLOGY, vol. 32, no. 5, 27 April 2017 (2017-04-27) , pages 992-1002, XP055570283, ISSN: 0815-9319, DOI: 10.1111/jgh.13627
- FOELL D ET AL: "Monitoring disease activity by stool analyses: from occult blood to molecular markers of intestinal inflammation and damage.", GUT JUN 2009, vol. 58, no. 6, June 2009 (2009-06), pages 859-868, ISSN: 1468-3288
- MICHAEL WAGNER: "Fecal markers of inflammation used as surrogate markers for treatment outcome in relapsing inflammatory bowel disease", WORLD JOURNAL OF GASTROENTEROLOGY, vol. 14, no. 36, 1 January 2008 (2008-01-01), page 5584, XP55413309, CN ISSN: 1007-9327, DOI: 10.3748/wjg.14.5584

## Description

### FIELD OF THE INVENTION

The present invention relates to methods for diagnosing inflammatory bowel disease (IBD), such as ulcerative colitis (UC) or Crohn's disease (CD) and assessing intestinal inflammation intensity by measuring the amount of an IBD-specific biomarker(s) in a sample of colorectal mucocellular layer non-invasively collected from the surface of the anal area of a human subject following the natural act of defaecation. Simultaneous quantitative evaluation of several biomarkers in samples of human colorectal mucocellular layer provides additional methods for IBD monitoring, assessing effectiveness of applied therapy and individually selecting specific therapeutic modalities for IBD patients. The invention also provides a method for distinguishing between UC and Crohn's disease.

### BACKGROUND OF THE INVENTION

Inflammatory bowel disease (IBD) is a group of common chronic disorders involving bowel inflammation. Ulcerative colitis (UC) and Crohn's disease (CD) are the most important conditions of this group. IBD is usually diagnosed in young adults. In most cases it is characterized by long remissions and incidental disabling flare-ups usually requiring treatment.

Currently there are about 2.4 million IBD patients in the EU and over 1.3 million in the US (Cosnes et al., 2011). The incidence and prevalence of the disease continue to grow (Molodecky et al, 2012). Once diagnosed, all IBD patients should be monitored for a possible relapse. Those developing relapses are treated, and treatment efficiency assessment is an important task in need of serious improvements. Differentiation between IBD and non-inflammatory diarrhoeas, such as Irritable Bowel Syndrome (IBS) constitutes another major problem, especially given that IBS affects 10-15% of the adult population in most developed Western countries (Agarwal & Whorwell, 2006; Khan & Chang, 2010).

IBD diagnosis confirmation usually requires colonoscopy, an efficient diagnostic procedure that is, however, highly invasive, expensive and can sometimes cause dangerous complications. Repeated colonoscopies in IBD patients may be dangerous; therefore currently IBD activity and therapy efficiency are usually assessed by disease activity indexes based on severity of clinical manifestations and results of indirect laboratory analyses (Satsangi et al, 2006). Colonoscopy is also widely used to differentiate between IBD and common functional conditions such as IBS.

Development of biomarker-based non-invasive diagnostic tests for IBD is generally recognised as an urgent problem, solution of which can help in addressing multiple important endpoints comprising: a) primary diagnosis of IBD and its differentiation from other conditions; b) differentiation between UC and CD; c) complication risk assessment; d) distinction between active IBD and remission; e) assessment of colonic mucosa damage and healing; f) IBD relapse prediction; g) Prediction of response to specific therapy and treatment choice; h) therapy efficiency monitoring and therapeutic adjustments (Lewis, 2011). Recent progress in biomarker use for these endpoints is reflected in numerous scientific publications and abundant patent literature. The main approaches can be roughly divided into methods employing non-colonic tissue or body fluids (especially blood/serum) and those using materials obtained directly from the colon, such as stool, colonic biopsy samples, and colonic lavage. The existing prior art is briefly outlined below.

The role of genetic factors in the pathogenesis of both UC (Louis et al, 2009) and CD (Weersma et al, 2009; Tamboli et al, 2011) is well known. There is a group of patent documents describing genetic markers for IBD diagnosis, often presented as complex multimarker panels exemplified by a family of patents by Harris and Alsobrook (US Pat. Nos. 7,833,720; 7,833,721; 7,879,553; 7,923,544; 8,222,390; 8,227,589). Methods detecting single gene variants associated with IBD (WO03/052412) or specifically with CD (US Pat. Nos. 6,001,569; 6,534,263) were also proposed. Determination of IBD-related changes in the human gut microbiome is the main element of another genotyping-based technique (US Pat. App. No. 2013/0045874). Other relevant patents using genetic markers describe approaches targeting changes in microRNAs (WO94/21662; US Pat App. Nos. 2011/0117111; 2013/0143764) and various gene expression profiles (US Pat. Nos. 7,875,431; 8,257,923; US Pat. App. Nos. 2009/0155788; 2009/0186034; 2009/0311260; 2010/0267575; 2011/0082188; EP1462527). However, genetic approaches have not provided a clinically applicable method so far, being mostly confined to the prediction of Crohn's disease risk (Weersma et al, 2009) and assessment of pharmacogenetics of drugs used for its treatment (Roberts & Barclay, 2012). In addition to limited diagnostic efficiency, most of these methods are exceedingly technically complex.

The use of protein biomarkers defines another major group of original approaches to IBD diagnosis. Several existing patents describe diagnostic methods employing biomarkers of this type detectable in serum. Detection of perinuclear anti-neutrophil cytoplasmic antibodies (pANCA) associated with UC and CD-associated anti-saccharomyces cerevisiae antibodies (ASCA) constitutes an important component of most techniques using serum samples, however various additional markers were also considered (Peyrin-Biroulet et al, 2007). A number of multimarker methods proposed for human serum samples combines pANCA and/or ASCA with either multiple protein biomarkers of human or bacterial origin (US Pat. Nos. 6,218,129; 7,608,414 7,759,079; 7,873,479; 8,315,818; 8,445,215; 8,463,553; US Pat. App. Nos. 2006/0154276; 2010/0015156; 2010/0021455; 2010/0129838; 2010/0254971; 2010/0255513; WO2005/009339) or additional genetic markers (US Pat. App. Nos. 2011/0229471; 2012/0171672; 2013/0203053; 2013/0225439). Some basically similar techniques for IBD detection using serum samples do not include pANCA or ASCA, being focused on other bacterial (US Pat. Nos. 7,361,733; 7,993,865; 7,993,866; 7,993,867; 8,318,901; US Pat. App. Nos. 2007/0275424; 2011/0251100; WO2009/135257; WO2011/130546) or human (US Pat. No. 7,358,058; US Pat. App. No. 2009/0258848) proteins associated with gut inflammation, in particular inflammatory cytokines (US Pat. App. Nos. 2010/0316992; 2012/0258883; WO2012/037199). In addition to ASCA, antibodies to GP2 (a membrane glycoprotein known to be expressed in the exocrine part of the pancreas) have recently been suggested as a new marker for CD (Somma et al, 2013). Other proposed techniques are designed to detect diagnostically informative subtypes of circulating monocytes (WO2012/172347), specific T-cell-associated molecules (US Pat. No. 7,989,173) or goblet cell antigen elevated in UC patients (US Pat. App. No. 2008/0293625). In addition, methods determining oligosaccharide ratio changes in IgG (US Pat. No. 8,043,832), assessing complex metabolite (over 100 small molecules) profiles (US Pat. App. No. 2012/0003158) or measuring antibodies against a range of dietary components (US Pat. App. No. 2012/0058497) were published. Although the outlined peripheral blood or serological marker panels may potentially be useful for differentiating UC from CD, disease monitoring and defining therapeutic strategies (Peyrin-Biroulet et al, 2007), they do not perform better than non-specific C-reactive protein (Palmon et al, 2008). None of them is currently applied for practical clinical use. Another major group of biomarker-based approaches in the area of IBD is related to analysing samples directly derived from the gastrointestinal tract. Prior art of this type deserves special attention since the present invention belongs to this group.

The idea of using colonic tissue for IBD testing could certainly be applied to invasively obtained tissue (biopsy) samples (US Pat. No. 7,972,807; Us Pat. App. Nos. 2004/132110; 2009/0305267), but stool sample analysis appears to be the most frequently used approach. A range of marker proteins detectable in stool samples obtained from IBD patients was investigated in this context (reviewed by Foell et al, 2009 and Lewis, 2011). The principal candidates were proteins found in neutrophil granules, in particular calprotectin, lactoferrin, S100A12 protein, dimeric pyruvate kinase, polymorphonuclear elastase, myeloperoxidase and human neutrophil lipocalin (Foell et al, 2009; Lewis, 2011; Sherwood, 2012). Among them calprotectin detection in stool samples using ELISA assay developed and patented by Fagerhol et al (US Pat. No. 5455160) was extensively investigated and provided the most consistent results (van Rheenen et al, 2010; Lewis, 2011). This calprotectin assay has recently been improved as described in US Pat. App. No. 2013/132347. A rapid calprotectin test for faecal samples has also been devised (WO2012/052586). Stool calprotectin quantification is the only biomarker-based test for IBD detection recommended for clinical use and currently employed by some clinicians (Sherwood, 2012).

Several patents by Boon et al describe IBD detection and differentiation from IBS using lactoferrin analysis in stool samples (US Pat. Nos. 7,192,724; 7,560,240; 7,892,762). The same group also proposed methods for distinguishing between UC and CD that employed detection in faeces of already mentioned ASCA (US Pat. No. 6872,540) or pANCA (US Pat. No. 7,736,858), alone or in combination with lactoferrin quantification (US Pat. No. 7,785,818). In other publications lactoferrin test was combined with neopterin detection (US Pat. App. No. 2012/0258477) or with assays for several protein biomarkers comprising calprotectin and a group of interleukins (US Pat. App. No. 2011/0212104). At the same time lactoferrin quantification in stool was also proposed for colorectal cancer diagnosis (US Pat. No. 5,552,292). In general IBD detection or differentiation between IBD and IBS using lactoferrin determination in faeces appeared to be less efficient than stool calprotectin assay (Sherwood, 2012).

Although information on S100A12 protein diagnostic performance for IBD detection is relatively scarce compared to calprotectin and lactoferrin, there are reports indicating that quantitative testing of stool samples for S100A12 may provide better results than calprotectin analysis (Kaiser et al, 2007), especially in children (de Jong et al, 2006; Sidler et al, 2008). Nevertheless, an attempt to use this marker for paediatric UC monitoring was not successful (Turner et al, 2010). In the absence of large clinical studies introduction of faecal S100A12 test into healthcare practice remains questionable (Sherwood, 2012). The lack of information on this biomarker is reflected in the available patent literature. The only relevant patent applications identified were US Pat. App. 2010/0311758 describing the use of S100A12 (alternatively called Calgranulin C) for diagnosing a wide range of inflammatory diseases and US Pat. App. No. 2009/0286328 proposing faecal S100A12 detection for colorectal cancer diagnosis. Dymeric pyruvate kinase (M2-PK), which was initially regarded as colorectal cancer marker detectable in stool samples (Hardt et al, 2004) has also emerged as a potential faecal marker for IBD (Jeffery et al, 2009; Turner et al, 2010). An ELISA assay for M2-PK is described in US Pat. No. 5,972,628 and its variant for the protein detection in stool samples in US Pat. No. 7,226,751. However, M2-PK is not applied in clinical practice.

Polymorphonuclear elastase is another enzyme present in neutrophils, which was proposed as a candidate IBD biomarker (Langhorst et al, 2008; Foell et al, 2009). Although an immunoassay for this protein exists (US Pat. No. 6,124,107), it is not regarded as a potential clinical test.

Some authors also suggested that inflammation-related neutrophil degranulation can be detected in stool samples by quantifying myeloperoxidase (Wagner et al, 2008; Masoodi et al, 2011) and human neutrophil lipocalin (Nielsen et al, 1996, 1999), but these tests are not sufficiently studied to be proposed for IBD detection.

Proteins associated with eosinophils, such as eosinophil cationic protein and eosin-derived neurotoxin (EDN) can also be detected in stool, but they were usually described as faecal markers of intestinal hypersensitivity and eosinophilic inflammation (Foell et al, 2009). The eosinophil-derived neurotoxin (EDN, also called Eosinophil Protein X) is a multifunctional protein possessing ribonuclease activity (Rosenberg, 2008). It is known to be a marker of eosinophil presence and degranulation, and its elevated amounts in stool samples were reported to correlate with allergic reactions (Majamaa et al, 1999; Magnusson et al, 2003). Although some authors described elevated EDN in stool being associated with the presence of inflammation (Bischoff et al, 1997; Saitoh et al, 1999; Peterson et al, 2002; Wagner et al, 2008), these observations were inconclusive. Increased EDN values were also reported in colorectal perfusion fluid (Carlson et al, 1999) and material collected from the surface of the rectal mucosa using an inflatable intrarectal device (Anderson et al, 2011; the collecting device was described in US Pat. App. 2008/0097238). The latter two studies, however, assessed very few IBD cases. In a patent by Gleich and Levy (US Pat. No. 5,928,883) EDN was proposed as one of eosinopil granule proteins (alongside eosinophil peroxidase), combined determination of which in whole gut lavage liquid could be used for IBD diagnosis. On the basis of the existing published evidence faecal EDN was not regarded as a promising biomarker of IBD, being less reliable than calprotectin or other stool biomarkers (Wagner et al, 2008; Foell et al, 2009). EDN was never considered as an IBD biomarker suitable for clinical use.

Several inflammatory cytokines were also proposed as potential biomarkers of IBD (Foell et al, 2009). Tumour necrosis factor alpha (TNFα), a small peptide predominantly produced by activated macrophages, could be a very good candidate, being a recognised therapeutic target in IBD patients (Danese et al, 2013). Although immunoassays for TNFα exist (e.g. US Pat. Nos. 5,223,395; 5,436,154; 7,285,269), the protein is unstable in stool samples (Foell et al, 2009). This constitutes a serious obstacle for using it for diagnostic purposes.

Additional biomarkers that are not derived from inflammatory cells can also be informative in the context of IBD diagnosis and monitoring. For example it is generally accepted that cell adhesion molecules (CAMs) are closely involved in leukocyte trafficking constituting a major mechanism in inflammatory process (Springer, 1995). Among them, intercellular adhesion molecule-1 (ICAM-1) is known to play an especially important role in the development of IBD inflammatory bowel disease (Vainer, 2010). Detection of a common polymorphism in the gene encoding ICAM-1 appears to correlate with IBD risk and was previously proposed as an approach to genetic screening for predisposition to IBD development (US Pat. Nos. 5,681,699; 6,008,335; 6,884,590). In addition, specific inhibition of ICAM-1 expression has recently been proposed as an approach to IBD treatment (Miner et al, 2006; Vainer, 2010). All available information regarding ICAM-1 presence in the colonic mucosa is limited by descriptive morphological observations from biopsy samples (Vainer, 2010), whereas it has never been quantified in either mucosal or stool samples.

Assessment of the degree of epithelial damage and its healing during convalescence constitutes another important aspect in the context of IBD. Soluble cytokeratin 18 (CK-18) is known to be released from epithelial cells following their death (Ueno et al, 2005). Although the presence of CK-18 in stool samples has never been investigated, elevated levels of this protein were once reported in samples obtained intrarectally from a few IBD patients (Anderson et al, 2011).

D-dimer is a small protein fragment generated during cross-linked fibrin degradation (Pabinger & Ay, 2009). Its increased presence can indicate chronic bleeding that is a common phenomenon in many IBD patients. Increased D-dimer levels in plasma samples from IBD patients were previously observed (Kume et al, 2007), but little was known on D-dimer changes in the gut. This biomarker could also be measured in intrarectally collected material (Anderson et al, 2011). D-dimer measurement might be informative for assessing intestinal inflammation severity and bleeding-related complication risk.

Some authors proposed using measurements of total human DNA in stool for IBD monitoring (Casellas et al, 2007), however the efficiency of this approach needs further evaluation. Finally, patent literature search allowed identifying less promising stool tests based upon the determination of intestinal O-glycans (US Pat. App. No. 2009/0311707), HMGB1 protein (US Pat. App. No. 2013/0137123) and COX-2 protein (US Pat. No. 7,220,825).

The presented background information shows that despite the availability of a number of potentially promising biomarkers of intestinal inflammation the field is still poorly developed. The only clinically employed biomarker-based test for IBD is stool calprotectin detection, the applicability of which is considerably limited due to the necessity of stool collection and handling. Presently there is no reliable alternative non-invasive test for IBD.

We have previously devised a new method of non-invasive collection of excreted colonic mucocellular layer (Loktionov, 2007) material from the anal area following natural bowel opening (WO2012/150453). This simple procedure described in WO2012/150453 is based on sample self-collection and provides material containing highly informative cells in abundance that can be easily applied to a range of biomarker detection-based diagnostic and monitoring applications in the area of colorectal disease. We have applied the new collection technique and tested a range of potential biomarkers in samples obtained from IBD patients and controls.

For convenience, a list of references cited herein follows:

### U.S. PATENT DOCUMENTS

| | | | |
|---|---|---|---|
| 4,816,567 | 03/1989 | Cabilly et al. | |
| 5,223,395 | 06/1993 | Gero | |
| 5,436,154 | 07/1995 | Barbanti et al. | |
| 5,455,160 | 10/1995 | Fagerhol et al. | |
| 5,545,806 | 08/1996 | Lonberg et al. | |
| 5,552,292 | 09/1996 | Uchida et al. | |
| 5,569,825 | 10/1996 | Lonberg et al. | |
| 5,625,126 | 04/1997 | Lonberg et al. | |
| 5,633,425 | 05/1997 | Lonberg et al. | |
| 5,661,016 | 07/1997 | Lonberg et al. 5,681,699 | 10/1997 Rotter et al. |
| 5,928,883 | 07/1999 | Gleich et al. | |
| 5,972,628 | 10/1999 | Eigenbrodt et al. | |
| 6,001,569 | 12/1999 | Plevy et al. | |
| 6,008,335 | 12/1999 | Rotter et al. | |
| 6,124,107 | 09/2000 | Humes et al. | |
| 6,187,546 | 02/2001 | O'Neill et al. | |
| 6,218,129 | 04/2001 | Walsh et al. | |
| 6,534,263 | 03/2003 | Plevy et al. | |
| 6,872,540 | 03/2005 | Boone et al. | |
| 6,884,590 | 04/2005 | Rotter et al. | |
| 7,192,724 | 03/2007 | Boone et al. | |
| 7,220,825 | 05/2007 | Nair | |
| 7,226,751 | 06/2007 | Eigenbrodt et al. | |
| 7,285,269 | 10/2007 | Babcock et al. | |
| 7,358,058 | 04/2008 | Bergmann et al. | |
| 7,361,733 | 04/2008 | Hershberg et al. | |
| 7,560,240 | 07/2009 | Boone et al. | |
| 7,608,414 | 10/2009 | Dotan et al. | |
| 7,736,858 | 06/2010 | Boone et al. | |
| 7,759,079 | 07/2010 | Oh et al. | |
| 7,785,818 | 08/2010 | Boone et al. | |
| 7,833,720 | 11/2010 | Harris et al. | |
| 7,833,721 | 11/2010 | Harris et al. | |
| 7,873,479 | 01/2011 | Lois et al. | |
| 7,875,431 | 01/2011 | Diehl et al. | |
| 7,879,553 | 02/2011 | Harris et al. | |
| 7,892,762 | 02/2011 | Boone et al. | |
| 7,923,544 | 04/2011 | Harris et al. | |
| 7,972,807 | 07/2011 | Phanstiel et al. | |
| 7,989,173 | 08/2011 | Chen | |
| 7,993,865 | 08/2011 | Targan et al. | |
| 7,993,866 | 08/2011 | Targan et al. | |
| 7,993,867 | 08/2011 | Targan et al. | |
| 8,043,832 | 10/2011 | Miyoshi et al. | |
| 8,222,390 | 07/2012 | Harris et al. | |
| 8,227,589 | 07/2012 | Harris et al. | |
| 8,257,923 | 09/2012 | Diehl et al. | |
| 8,315,818 | 11/2012 | Lois et al. | |
| 8,317,728 | 11/2012 | Triva | |
| 8,318,901 | 11/2012 | Hershberg et al. | |
| 8,445,215 | 05/2013 | Wang et al. | |
| 8,463,553 | 06/2013 | Lois et al. | |

### U.S. PATENT APPLICATIONS

| | | |
|---|---|---|
| US2004/0132110 | 07/2004 | Desreumaux et al. |
| US2006/0154276 | 07/2006 | Lois et al. |
| US2007/0275424 | 11/2007 | Gewirtz et al. |
| US2008/0097238 | 04/2008 | Loktionov et al. |
| US2008/0293625 | 11/2008 | Stocker et al. |
| US2009/0155788 | 06/2009 | Abbas et al. |
| US2009/0186034 | 07/2009 | Abbas et al. |
| US2009/0258848 | 10/2009 | Chakravarti et al. |
| US2009/0286328 | 11/2009 | Wild et al. |
| US2009/0305267 | 12/2009 | Krause et al. |
| US2009/0311260 | 12/2009 | Goddard et al. |
| US2009/0311707 | 12/2009 | Xia |
| US2010/0015156 | 01/2010 | Dubinsky et al. |
| US2010/0021455 | 01/2010 | Targan et al. |
| US2010/0129838 | 05/2010 | Barken |
| US2010/0254971 | 10/2010 | Dotan et al. |
| US2010/0255513 | 10/2010 | Denson et al. |
| US2010/0267575 | 10/2010 | Xu et al. |
| US2010/0311758 | 12/2010 | Roth et al. |
| US2010/0316992 | 12/2010 | Debad et al. |
| US2011/0082188 | 04/2011 | Chakravarti |
| US2011/0117111 | 05/2011 | Kwon et al. |
| US2011/0212104 | 09/2011 | Beaumont et al. |
| US2011/0229471 | 09/2011 | Rotter et al. |
| US2011/0251100 | 10/2011 | Li et al. |
| US2012/0003158 | 01/2012 | Alexander et al. |
| US2012/0058497 | 03/2012 | Suga et al. |
| US2012/0171672 | 07/2012 | Barken et al. |
| US2012/0258477 | 10/2012 | Buchman |
| US2012/0258883 | 10/2012 | Chappell et al. |
| US2013/0045874 | 02/2013 | Ehrlich |
| US2013/0137123 | 05/2013 | Cucchiara et al. |
| US2013/0143764 | 06/2013 | Ogier-Denis et al. |
| US2013/0203053 | 08/2013 | Princen et al. |
| US2013/0225439 | 08/2013 | Princen et al. |
| US2013/132347 | 09/2013 | Mørk et al. |

### OTHER PATENT DOCUMENTS

| | | |
|---|---|---|
| WO94/04690 | 03/1994 | Ashkenazi et al |
| WO94/21662 | 09/1994 | Altman et al. |
| WO97/34631 | 09/1997 | Ward |
| WO03/052412 | 06/2003 | Allen et al. |
| EP1462527 | 09/2004 | Costello et al. |
| WO2009/135257 | 11/2009 | Radford-Smith |
| WO2011/130546 | 10/2011 | Norman |
| WO2012/037199 | 03/2012 | Li et al. |
| WO2012/052586 | 04/2012 | Genzor et al. |
| WO2012/150453 | 07/2012 | Loktionov et al. |
| WO2012/172347 | 12/2012 | Winqvist et al. |

### OTHER PUBLICATIONS:

Agarwal & Whorwell (2006) BMJ 332: 280-283.
Anderson et al. (2011) Int J Colorectal Dis 26: 1287-1297.
Bandaletova et al. (2002) APMIS 110 : 239-246.
Bischoff et al. (1997) Dig Dis Sci 42: 394-403.
Carlson et al. (1999) Am J Gastroenterol 94: 1876-83.
Carter et al. (1992) Nat Biotechnol 10: 163-167.
Carter et al. (1995) J Hematotherapy 4 : 463-470.
Casellas et al. (2007) Inflamm Bowel Dis 13: 386-390.
Clackson et al. (1991) Nature 352 : 624-628.
Cole et al. (1985) in Monoclonal Antibodies and Cancer Therapy, Alan R.Liss, Inc. NY: pp. 77-96.
Cosnes et al. (2011) Gastroenterology 140: 1785-1794.
Danese et al. (2013) Aliment Pharmacol Ther 37 : 855-866.
De Jong et al. (2006) Inflamm Bowel Dis 12: 566-572.
Foell et al. (2009) Gut 58: 859-868.
Hardt et al. (2004) Br J Cancer 91 : 980-984.
Holliger & Hudson (2005) Nat Biotechnol 23 : 1126-1136.
Jefferey et al. (2009) Inflamm Bowel Dis 15: 1630-1634.
Kabat (1980) J Immunol 125 : 961-969.
Kabat et al (1992) Sequences of Proteins of Immunological Interest. 5-th edition. NIH,Bthesda, MD: 2719p.
Kaiser et al. (2007) Gut 56: 1706-1713.
Khan & Chang (2010) Nat Rev Gastroenterol Hepatol 7: 565-581.
Kohler & Milstein (1995) Nature 256: 495-497.
Kozbor & Roder (1983) Immunol Today 4: 72-79.
Langhorst et al. (2008) Am J Gastroenterol 103: 162-169.
Lewis (2011) Gastroenterology 140: 1817-1826.
Linnet (1985) Clin Chem 31: 574-580
Loktionov (2007) Int J Cancer 120: 2281-2289.
Loktionov et al. (1998) Clin Ca Res 4 : 337-342.
Loktionov et al. (2009) Int J Oncol 34 : 301-311.
Loktionov et al. (2010) Int J Cancer 126 : 1910-1919.
Lonberg & Huszar (1995) Int Rev Immunol 13 : 65-93.
Louis et al. (2009) Gut 58: 1173-1176.
Magnusson et al. (2003) Clin Exp Allergy 33: 1052-1059.
Majamaa et al. (1999) Clin Exp Allergy 29: 1502-1506.
Marks et al. (1991) J Mol Biol 222 : 581-597.
Masoodi et al. (2011) Ger Med Sci 9: Doc3.
Merchant et al. (1998) Nat Biotechnol 16: 677-681.
Miner et al. (2006) Aliment Pharmacol Ther 23: 1403-1413.
Molodecky et al. (2012) Gastroenterology 142: 46-54.
Nielsen et al. (1996) Gut 38: 414-420.
Nielsen et al. (1999) Am J Gastroenterol 94: 2923-2928.
Pabunger & Ay (2009) Atheroscl Thromb Vasc Biol 29: 332-336.
Palmon et al. (2008) Inflamm Bowel Dis 14: S187-S189.
Peterson et al. (2002) Am J Gastroenterol 97: 1755-1762.
Peyrin-Biroulet et al. (2007) Inflamm Bowel Dis 13: 1561-1566.
Roberts & Barclay (2012) J Gastroenterol Hepatol 27: 1546-1554.
Rodrigues et al. (1993) J Immunol 151: 6954-6961.
Rosenberg (2008) Curr Pharm Biotechnol 9: 135-140.
Saitoh et al. (1999) Am J Gastroenterol 94: 3513-3520.
Satsangi et al. (2006) Gut 55: 749-753.
Sherwood (2012) J Clin Pathol 65: 981-985.
Sidler et al. (2008) Inflamm Bowel Dis 14: 359-366.
Somma et al. (2013) Gastroenterol Res Pract 2013: 683824.
Springer (1995) Annu Rev Physiol 57: 827-872.
Suresh et al. (1986) Methods Enzymol 121: 210-228.
Tamboli et al. (2011) Clin Experim Gastroenterol 4 : 127-140.
Turner et al. (2010) Gut 59: 1207-1212.
Ueno et al. (2005) Biomed Pharmacother 59: S359-S362.
Vainer (2010) APMIS 118 (Suppl 129): 1-46.
van Rheenen et al. (2010) BMJ 341: c3369.
Wagner et al. (2008) World J Gastroenterol 14: 5584-5589.
Weersma et al. (2009) Gut 58: 388-395.

### SUMMARY OF THE INVENTION

In a first aspect of the invention there is provided a method for diagnosing an inflammatory bowel disease (IBD), the method comprising: determining the concentration of at least one IBD-specific biomarker, or a fragment or variant thereof, in a sample of the colonic mucocellular layer obtained from a subject, wherein the sample is obtained from the surface of the anal area following defaecation by taking a swab of said area; comparing said concentration to a threshold value; and determining that the subject has IBD when the concentration of at least one IBD-specific biomarker is said sample is equal to or greater than the threshold value; wherein the at least one IBD-specific biomarker is eosinophil-derived neurotoxin (EDN); and wherein the threshold value is between 15ng/ml and 35 ng/ml when the collected sample is lysed in 3ml of lysis buffer.

Alternatively, the sample is an intestinal mucocellular layer. Alternatively, the sample may be a sample originating from the internal surface of the bowel.

In a preferred embodiment, the sample is taken from the anal area in the vicinity of the exterior opening of the anal canal.

We describes that the at least one IBD-specific biomarker may selected from the group consisting of eosinophil-derived neurotoxin (EDN), calprotectin, S100A12, ICAM1, CK-18, D-dimer, TNF-α, ASCA, pANCA, anti-GP2 antibodies, lactoferrin and total amount of human DNA in the sample or a combination thereof. The IBD-specific biomarker may also be a cytokine, alone or in combination with another IBD-specific biomarker.

In one embodiment, the IBD-specific biomarker is EDN and the threshold value is (or is equivalent to) between 15ng/ml and 35 ng/ml when the (collected) sample is lysed in 3ml of lysis buffer. Alternatively, the IBD-specific biomarker is calprotectin and the threshold value is or is equivalent to between 3.5µg/ml and 6.0 µg/ml when the collected sample is lysed in 3ml of lysis buffer. As a further alternative, the IBD-specific biomarker is S100A12 and the threshold value is or is equivalent to 50ng/ml when the collected sample is lysed in 3ml of lysis buffer. As another alternative, the IBD-specific biomarker is ICAM1 and the threshold value is or is equivalent to 150pg/ml when the collected sample is lysed in 3ml of lysis buffer.

In an another embodiment, the IBD-specific biomarker is EDN the threshold value is, or is equivalent to, 15ng/ml when the collected sample is lysed in 3ml of lysis buffer. Alternatively, the IBD-specific biomarker is calprotectin and the threshold value is, or is equivalent to, 3.5 µg/ml when the collected sample is lysed in 3ml of lysis buffer.

In one embodiment the concentration of the IBD-specific biomarker is determined by contacting the sample with at least one antibody capable of specifically binding the IBD-specific biomarker or a fragment or variant thereof.

We also describe a method for diagnosing an inflammatory bowel disease (IBD), the method comprising determining the concentration of the calprotectin protein, or a fragment or variant thereof, in a sample of the colonic mucocellular layer obtained from a subject and determining that the subject has IBD when the concentration of calprotectin is, or is equivalent to a value, equal to or greater than 3.5 µg/ml when the collected sample is lysed in 3ml of lysis buffer.

In one embodiment of the methods described herein, the method comprises determining that the subject has IBD when the concentration of EDN is, or is equivalent to a value, equal to or greater than 24ng/ml when the collected sample is lysed in 3ml of lysis buffer. Alternatively, the concentration of EDN is, or is equivalent to a value, equal to or greater than 35ng/ml when the collected sample is lysed in 3ml of lysis buffer. In a further alternative embodiment, the concentration of EDN is, or is equivalent to a value, equal to or greater than a concentration value within the range 15ng/ml to 35ng/ml when the collected sample is lysed in 3ml of lysis buffer.

In another aspect of the methods described herein, the method comprises determining that the subject has IBD when the concentration of calprotectin is, or is equivalent to a value, equal to or more than 4.7 µg/ml when the collected sample is lysed in 3ml of lysis buffer. Alternatively, the concentration of calprotectin is, or is equivalent to a value, equal to or more than 6.0 µg/ml when the collected sample is lysed in 3ml of lysis buffer. In a further alternative , the concentration of calprotectin is ,or is equivalent to a value, equal to or greater than a concentration value within the range 3.5µg/ml and 6.0 µg/ml when the collected sample is lysed in 3ml of lysis buffer.

In a fourth aspect of the invention there is provided a method for monitoring the effectiveness of a treatment for IBD the method comprising determining the concentration of at least one IBD-specific biomarker, or a fragment or variant thereof, in a sample of the colonic mucocellular layer obtained from a subject, wherein the sample is obtained from the surface of the anal area following defaecation, comparing said concentration to a threshold value and determining that the treatment is effective when the concentration of at least one IBD-specific biomarker is said sample is less than the threshold value.

In one embodiment the IBD-specific biomarker is EDN the threshold value is, or is equivalent to, between 35-120ng/ml when the collected sample is lysed in 3ml of lysis buffer. Alternatively, the threshold value is, or is equivalent to, less than 100ng/ml when the collected sample is lysed in 3ml of lysis buffer.

In an alternative aspect, the IBD-specific biomarker is calprotectin and the threshold value is, or is equivalent to, between 6.0 and 10.0 µg/ml when the collected sample is lysed in 3ml of lysis buffer. In a further example the threshold value is, or is equivalent to a value, less than 7.5 µg/ml when the collected sample is lysed in 3ml of lysis buffer.

We also describe a method for monitoring the effectiveness of a treatment for IBD, the method comprising determining the concentration of the EDN protein, or a fragment or variant thereof, in a sample of the colonic mucocellular layer obtained from a subject post-treatment, and determining that the treatment is effective when the concentration of EDN is ,or is equivalent to, less than a value between 35-120ng/ml, when the collected sample is lysed in 3ml of lysis buffer. Preferably, the concentration of the EDN protein, or a fragment or variant thereof is measured at at least two time points post-treatment and the method comprises determining that the treatment is effective when the concentration of EDN at at least one time point (preferably the later time point) is ,or is equivalent to, less than a value between 35-120ng/ml, when the collected sample is lysed in 3ml of lysis buffer. Alternatively, the concentration of EDN is, or is equivalent to, less than 100ng/ml when the collected sample is lysed in 3ml of lysis buffer.

We also describe a method for monitoring the effectiveness of a treatment for IBD, the method comprising determining the concentration of the calprotectin protein, or a fragment or variant thereof, in a sample of the colonic mucocellular layer obtained from a subject post-treatment, and determining that the treatment is effective when the concentration of calprotectin is ,or is equivalent to, less than a value between 6.0 and 10.0 µg/ml when the collected sample is lysed in 3ml of lysis buffer. Preferably the concentration of the calprotectin protein, or a fragment or variant thereof is measured at at least two time points post-treatment and the method comprises determining that the treatment is effective when the concentration of calprotectin at at least one time point (preferably the later time point) is,or is equivalent to, less than a value between 6.0 and 10.0 µg/ml when the collected sample is lysed in 3ml of lysis buffer. Alternatively, the concentration of calprotectin is, or is equivalent to a value, less than 7.5 µg/ml when the collected sample is lysed in 3ml of lysis buffer.

In a further alternative, monitoring the effectiveness of a treatment for IBD comprises determining the concentration of the EDN protein and/or calprotectin protein, or a fragment or variant thereof, in a sample of the colonic mucocellular layer obtained from a subject pre and at least one time point post-treatment and determining that the treatment is ineffective when the concentration of EDN and/or calprotectin in the at least one post-treatment sample is equivalent to or greater than the concentration in the pre-treatment sample. In a preferred embodiment the treatment is ineffective when the concentration in the post-treatment sample is ,or is equivalent to, a value equal to or greater than a value between 35-120ng/ml for EDN and between 6.0 and 10.0 µg/ml for calprotectin when the collected sample is lysed in 3ml of lysis buffer. In a preferred embodiment, the concentration of the EDN protein and/or calprotectin protein, or a fragment or variant thereof is measured at at least two time points post-treatment and the method comprises determining that the treatment is ineffective when the concentration of EDN and/or calprotectin is ,or is equivalent to a value, equal to or greater than a value between 35-120ng/ml for EDN and between 6.0 and 10.0 µg/ml for calprotectin. In a further alternative, the concentration in the post-treatment sample is, or is equivalent to, a value equal to or greater than 100 ng/ml for EDN and 7.5 µg/ml for calprotectin when the collected sample is lysed in 3ml of lysis buffer.

In one embodiment, the concentration of EDN and/or calprotectin is measured at at least one of the following time points: 0 (pre-treatment), 10, 20, 30, 40, 50, 60, 90, 120, 240, 360 days post-treatment or a combination thereof. In one embodiment the concentration of EDN and/or calprotectin is measured at day 0 (pre-treatment) and any one or more of the following time points: 10, 20, 30, 40, 50, 60, 90, 120, 240, 360 days post-treatment.

IWe also describe a method for monitoring for disease relapse in an IBD patient in remission, the method comprising determining the concentration of at least one IBD-specific biomarker, or a fragment or variant thereof, in a sample of the colonic mucocellular layer obtained from a subject in remission, preferably at predetermined intervals, wherein the sample is obtained from the surface of the anal area following defaecation, comparing said concentration to a threshold value and determining a disease relapse when the concentration of the of at least one IBD-specific biomarker is said sample is equal to or greater than the threshold value.

In one embodiment the IBD-specific biomarker is EDN the threshold value is, or is equivalent to, between 35-120ng/ml, when the collected sample is lysed in 3ml of lysis buffer. Alternatively the threshold value is, or is equivalent to, 100ng/ml when the collected sample is lysed in 3ml of lysis buffer.

Alternatively, the IBD-specific biomarker is calprotectin and the threshold value is, or is equivalent to, between 6.0 and 10.0 µg/ml when the collected sample is lysed in 3ml of lysis buffer. In a further alternative, the threshold value is, or is equivalent to, 7.5 µg/ml when the collected sample is lysed in 3ml of lysis buffer.

In a further aspect of the invention there is provided a method for monitoring for disease relapse in an IBD patient in remission, the method comprising determining the concentration of the EDN protein, or a fragment or variant thereof, in a sample of the colonic mucocellular layer obtained from a subject at, preferably, predetermined intervals, and determining a disease relapse when the concentration of EDN is ,or is equivalent to, a value equal to or greater than a value between 35-120ng/ml when the collected sample is lysed in 3ml of lysis buffer. Preferably, the concentration of the EDN protein, or a fragment or variant thereof is measured at at least two time points and the method comprises determining a disease relapse when the concentration of EDN at at least one time point is ,or is equivalent to, a value equal to or greater than a value between 35-120ng/ml when the collected sample is lysed in 3ml of lysis buffer. In a further alternative, the concentration of EDN is, or is equivalent to, a value equal to or greater than 100ng/ml when the collected sample is lysed in 3ml of lysis buffer.

We also describe a method for monitoring for disease relapse in an IBD patient in remission, the method comprising determining the concentration of the calprotectin protein, or a fragment or variant thereof, in a sample of the colonic mucocellular layer obtained from a subject at predetermined intervals, and determining a disease relapse when the concentration of calprotectin is ,or is equivalent to, a value equal to or greater than a value between 6.0 and 10.0 µg/ml when the collected sample is lysed in 3ml of lysis buffer. Preferably, the concentration of the calprotectin protein, or a fragment or variant thereof, is measured at at least two time points and the method comprises determining a disease relapse when the concentration of calprotectin is ,or is equivalent to, a value equal to or greater than a value between 6.0 and 10.0 µg/ml when the collected sample is lysed in 3ml of lysis buffer. More preferably the concentration of calprotectin is, or is equivalent to, a value equal to or greater than 7.5 µg/ml when the collected sample is lysed in 3ml of lysis buffer.

IWe also describe a method of selecting a treatment for an IBD patient, the method comprising determining the concentration of at least one IBD-specific biomarker, or a fragment or variant thereof, in a sample of the colonic mucocellular layer obtained from a subject, wherein the sample is obtained from the surface of the anal area following defaecation, comparing said concentration to a threshold value and selecting a IBD-targeted therapy when the concentration of the at least one IBD-specific biomarker is said sample is equal to or greater than the threshold value.

Preferably the IBD-specific biomarker is EDN the threshold value is or is equivalent to, 35ng/ml when the collected sample is lysed in 3ml of lysis buffer. Alternatively, the IBD-specific biomarker is calprotectin and the threshold value is or is equivalent to, 6.0 µg/ml when the collected sample is lysed in 3ml of lysis buffer.

We also describe a method of selecting a treatment for an IBD patient, the method comprising determining the concentration of the EDN protein, or a fragment or variant thereof, in a sample of the colonic mucocellular layer obtained from a subject, and selecting a IBD-targeted therapy when the concentration of EDN is, or is equivalent to, a value, equal to or greater than a value between between 35-120ng/ml when the collected sample is lysed in 3ml of lysis buffer. Preferably, the concentration of EDN is, or is equivalent to, a value equal to or more than 100ng/ml when the collected sample is lysed in 3ml of lysis buffer.

We also describe a method of selecting a treatment for an IBD patient, the method comprising determining the concentration of the calprotectin protein, or a fragment or variant thereof, in a sample of the colonic mucocellular layer obtained from a subject, and selecting a IBD-targeted therapy when the concentration of calprotectin is , or is equivalent to, a value, equal to or greater than a value between 6.0 and 10.0 µg/ml when the collected sample is lysed in 3ml of lysis buffer. Preferably, the concentration of calprotectin is, or is equivalent to, a value equal to or greater than 7.5 µg/ml when the collected sample is lysed in 3ml of lysis buffer.

We further describe a method of selecting a subject for IBD-targeted therapy, the method comprising determining the concentration of at least one IBD-specific biomarker, or a fragment or variant thereof, in a sample of the colonic mucocellular layer obtained from a subject, wherein the sample is obtained from the surface of the anal area following defaecation, comparing said concentration to a threshold value and selecting the subject for IBD-targeted therapy when the concentration of the at least one IBD-specific biomarker is said sample is equal to or greater than the threshold value.

Preferably the IBD-specific biomarker is EDN the threshold value is, or is equivalent to, 35ng/ml when the collected sample is lysed in 3ml of lysis buffer. Alternatively, the IBD-specific biomarker is calprotectin and the threshold value is, or is equivalent to, 6.0 µg/ml when the collected sample is lysed in 3ml of lysis buffer.

We also describe a method of selecting a subject for IBD-targeted therapy, the method comprising determining the concentration of the EDN protein, or a fragment or variant thereof, in a sample of the colonic mucocellular layer obtained from the subject, and selecting the subject for IBD-targeted therapy when the concentration of EDN is, or is equivalent to, a value, equal to or greater than a value between 35-120ng/ml when the collected sample is lysed in 3ml of lysis buffer. Preferably, the concentration of EDN is, or is equivalent to, a value equal to or more than 100ng/ml when the collected sample is lysed in 3ml of lysis buffer.

We also describe a method of selecting a subject for IBD-targeted therapy, the method comprising determining the concentration of the calprotectin protein, or a fragment or variant thereof, in a sample of the colonic mucocellular layer obtained from the subject, and selecting the subject for IBD-targeted therapy when the concentration of calprotectin is, or is equivalent to, a value, equal to or greater than a value between 6.0 and 10.0 µg/ml when the collected sample is lysed in 3ml of lysis buffer. Preferably, the concentration of calprotectin is, or is equivalent to, a value equal to or more than 7.5 µg/ml when the collected sample is lysed in 3ml of lysis buffer.

Preferably, the IBD is Crohn's disease. Alternatively the IBD is Ulcerative colitis.

Preferably the methods further comprise determining the concentration of at least one further biomarker for IBD in said sample. Preferably, the at least one further biomarker is selected from the group consisting of S100A12, ICAM1, CK-18, D-dimer, TNF-α, ASCA, pANCA, anti-GP2 antibodies, lactoferrin and total amount of human DNA in the sample or a combination thereof. The IBD-specific biomarker may also be a cytokine, alone or in combination with another IBD-specific biomarker.

The method may further comprise analysing the cytology of said sample.

In one aspect, the subject has or is at risk of developing IBD. Alternatively, the subject has no clinical signs or manifestations of IBD (i.e. the subject is asymptomatic). Preferably, the subject is human.

In a further aspect, the concentration of the IBD-specific biomarker is determined by contacting the sample with at least one antibody capable of specifically binding the IBD-specific biomarker protein, or a fragment or variant thereof. Alternatively, the concentration of the IBD-specific biomarker is determined by detecting the expression levels of the IBD-specific biomarker, or a fragment or variant thereof. In a further aspect total amount of human DNA in the sample is determined. The methods may further comprise comparing the concentration of the IBD-specific biomarker in a subject's sample with the concentration levels in a control or reference sample.

We also describe a method for diagnosing an IBD, the method comprising determining the concentration of both EDN and calprotectin , or a fragment or variant thereof of either protein, in a sample of colonic mucocellular layer obtained from a subject; comparing the concentration of both proteins to a threshold value to obtain a ratio of calprotectin concentration/calprotectin threshold and EDN concentration/EDN threshold; and determining that the subject has IBD when either or both ratio is above 1.0.

Preferably, the threshold value for calprotectin is, or is equivalent to a value, of 4.7 µg/ml when the collected sample is lysed in 3ml of lysis buffer. In a further aspect, the threshold value for calprotectin is ,or is equivalent to a value, of 3.5 µg/ml when the collected sample is lysed in 3ml of lysis buffer. Alternatively, the threshold value for calprotectin is ,or is equivalent to a value, 6.0 µg/ml when the collected sample is lysed in 3ml of lysis buffer.

In embodiment further alternative, the threshold value for EDN is, or is equivalent to a value, of 24ng/ml when the collected sample is lysed in 3ml of lysis buffer. Preferably, the threshold value for EDN is 15ng/ml when the collected sample is lysed in 3ml of lysis buffer. Alternatively the threshold value for EDN is 35ng/ml when the collected sample is lysed in 3ml of lysis buffer.

Preferably, the IBD is ulcerative colitis. Alternatively, the IBD is Crohn's disease.

In a further aspect, the method further comprises determining the concentration of at least one further biomarker for IBD in said sample. In one aspect the at least one further biomarker is selected from the group consisting of S100A12, ICAM1, CK-18, D-dimer, TNF-α, ASCA, pANCA, anti-GP2 antibodies, lactoferrin and total amount of human DNA in the sample or a combination thereof. The IBD-specific biomarker may also be a cytokine, alone or in combination with another IBD-specific biomarker.

In a further aspect of the invention there is provided a method of differentially diagnosing ulcerative colitis from Crohn's disease, the method comprising determining the concentration of both calprotectin and EDN, or a fragment or variant thereof of either protein, in a sample of colonic mucocellular layer obtained from a subject, comparing the concentration of both proteins to a threshold value to obtain a ratio of calprotectin concentration/calprotectin threshold and EDN concentration/EDN threshold; and determining that the subject has ulcerative colitis when either or both ratio is above 4.0. The method may also further comprise determining the concentration of at least one other IBD-specific biomarker. Preferably, the at least one other IBD-specific biomarker is ASCA and/or GP-2 antibodies.

In a preferred embodiment, the method further comprises determining the concentration of the ICAM1 protein, or a fragment or variant thereof in said sample. Preferably, the method further comprises multiplying the ratio by a weighting factor when ICAM1 cannot be detected in said sample. Preferably, the weighting factor is less than zero. More preferably, the weighting factor is 0.1. In one example,, the subject is determined to have ulcerative colitis (rather than Crohn's disease) when either or both ratio is equal to or greater than 4.0. In a further preferred embodiment, the subject is determined to have ulcerative colitis when either or both ratio is equal to or greater than 9.0.

The method may further comprise determining the concentration of at least one further IBD-specific biomarker in said sample. The at least one further IBD-specific biomarker may be selected from the group consisting of ASCA (Anti-Saccharomyces cerevisiae antibodies), GP-2 and perinuclear cytoplasmic antibodies (pANCA).

We also describe a method for diagnosing ulcerative colitis, the method comprising determining the concentration of the S100A12 protein, or a fragment or variant thereof, in a sample of colonic mucocellular layer obtained from a subject, and determining that the subject has ulcerative colitis when the concentration of S100A12 is,or is equivalent to a value, equal or more than 50 ng/ml when the collected sample is lysed in 3ml of lysis buffer.

We also describe a method for diagnosing an inflammatory bowel disease (IBD), the method comprising determining the concentration of the ICAM1 protein, or a fragment or variant thereof, in a sample of the colonic mucocellular layer obtained from a subject, comparing said concentration to a threshold value and determining that the subject has an IBD when the concentration of ICAM1 is equal to or greater than the threshold value. In one example the threshold value is or is equivalent to 150pg/ml when the collected sample is lysed in 3ml of lysis buffer.

We further describe a method of differentially diagnosing ulcerative colitis from Crohn's disease, the method comprising determining the concentration of ICAM1, or a fragment or variant thereof in a sample of colonic mucocellular layer obtained from a subject, comparing the concentration to a threshold value and determining that the subject has ulcerative colitis when the concentration of ICAM1 is equal to or greater than the threshold vale. In one example the threshold value is or is equivalent to 150pg/ml when the collected sample is lysed in 3ml of lysis buffer.

We also describe a method of selecting a treatment for an IBD patient, the method comprising determining the concentration of the ICAM1 protein, or a fragment or variant thereof, in a sample of the colonic mucocellular layer obtained from a subject, and selecting a ICAM 1-targeted therapy when the concentration of ICAM1 is above a threshold value. In one example the threshold value is or is equivalent to 150pg/ml when the collected sample is lysed in 3ml of lysis buffer. In one embodiment the ICAM1-targeted therapy is alicaforsen.

We further describe a method for assessing colonic mucosa damage, the method comprising determining the concentration of at least one epithelial-damage specific biomarker, or a fragment or variant thereof, in a sample of the colonic mucocellular layer obtained from a subject, comparing said concentration to a threshold value, and determining that the colonic mucosa is damaged when the concentration of the at least one epithelial-damage specific biomarker is equal to or greater than the threshold value. In one example the epithelial damage-specific biomarker is CK-18 and the threshold value is, or is equivalent to 300 U/L when the collected sample is lysed in 3ml of lysis buffer. In a further example, the epithelial damage-specific biomarker is CK-18 and the threshold value is, or is equivalent to 500 U/L when the collected sample is lysed in 3ml of lysis buffer. In another example the epithelial damage-specific biomarker is CK-18 and the threshold value is, or is equivalent to, a value within the range 100 U/L to 35 U/L when the collected sample is lysed in 3ml of lysis buffer.

In an alternative aspectof the methods described above the threshold value is determined by measuring the concentration of an IBD-specific biomarker in a group of one or more control subjects. Preferably, a group of five or more control subjects. More preferably, a group of ten or more control subjects.

Alternatively the threshold value defines a fractile of a distribution of measured concentrations for a population of control subjects without IBD. Preferably, said fractile is a 0.9 or greater fractile. Preferably, said distribution is assumed to be a Gaussian distribution.

Alternatively, the threshold value identifies that said subject is IBD-positive with greater than a threshold value. In a further alternative embodiment, said threshold value is associated with a defined probability of determining that said subject has IBD, when said subject has IBD. Preferably, said defined probability is greater than 60%, preferably 65%, preferably 70%, preferably 75%, preferably 80%, preferably 85%, preferably 90%, preferably 95%, preferably 100%.

In one embodiment of any of the methods described above the sample of colonic mucocellular layer from said subject is obtained from the surface of the anal area following defaecation. Preferably, the sample is obtained from the surface of the anal area in the vicinity of the exterior opening of the anal canal. The sample may be obtained by taking a swab of said area. Preferably the sample is taken prior to cleaning the area. Preferably, the sample is taken within 5 minutes of defaeccation. More preferably, the sample is taken within 4, 3, 2 or 1 minute of defaecation.

In embodiments of the invention, when the concentration of a biomarker, in particular EDN and/or calprotectin is determined, it may be determined in any volume of lysis, in which case the measured concentration may be expressed as if the lysis had been in 3ml of solution, i.e. normalised to the above-mentioned 3ml of lysis buffer.

For reasons of clinical repeatability of the test it can also be advantageous to define the threshold with reference to a standardised swab size/type and/or swab procedure - but this is not essential to the above-mentioned threshold values used, for example, for distinguishing between patient groups. This is because although it may be useful to have clinical tests conducted under standardised conditions, in practice the variation introduced by say, using one swab as compared with multiple swabs of the same region, or by using a larger or a smaller swab on a region, may be relatively small. Nonetheless such a standardised swab procedure may comprise, for example, a single swab of the target area and/or a defined swab size and/or shape. For example the swab shape may be defined as a circle or oval, optionally with a minimum dimension of not less than 5mm and/or with a maximum dimension of not more than 10mm, 15mm or 20mm. Additionally or alternatively the swab size may be defined as having a surface area of at least 40mm², 160mm², 350mm² or 600mm².

We also describe a non-invasive method for collecting a sample of intestinal or bowel cells or cell fragments comprising taking a swab of mucocellular layer material originating from said bowel or intestine from the surface of the anal area in the vicinity of the exterior opening of the anal canal, wherein said swab is taken following defaecation. Preferably the swab is taken prior to cleaning the area. In a further example, we describe a method of collecting a sample of intestinal or bowel cells or cell fragments using the above method and analysing said sample for the presence of one or more IBD-specific markers. Accordingly, we also provide a method for diagnosing and screening for bowel disease using the above method. The bowel disease may be a IBD, colorectal cancer, anal cancer or advanced colorectal polyps.

Finally, we describe a kit. Preferably the kit is suitable for implementing any of the above described methods. In one example the kit comprises at least one antibody, wherein each of said antibody is capable of binding to at least one IBD-specific biomarker or a fragment or variant thereof. Preferably, the antibody is capable of binding to EDN and/or calprotectin or a fragment or variant thereof. Alternatively, the kit comprises at least one antibody capable of binding S100A12, or a fragment or variant thereof. In a further alternative the kit comprises at least one antibody capable of binding ICAM1, or a fragment or variant thereof.

Preferably the kit comprises agents for the detection of the at least one antibody binding to said biomarker. In a further example the kit comprises instructions for use. In a further example the kit comprises a positive control sample.

Preferably, the kit comprises at least one swab. Preferably the kit comprises two swabs. In a further preferred aspect, the kit further comprises a sample tube. Preferably, the sample tube comprises a material-lysing lysis buffer. The sample tube may further comprise a material-preserving buffer. The kit may further comprise a fixative for cytological samples. The kit may further comprise a sampling card containing at least one, preferably two, microscope slides. In a further example the kit further comprises a lateral flow assay. Alternatively the kit comprises an electrochemical biosensor or biosensors.

In all above embodiments where a range is stated, the value may be either end-point of the range, or a value within the stated range. For example, where the threshold value is between 15ng/ml and 35 ng/ml, this value is of 15ng/ml and 35ng/ml.

### BRIEF DESCRIPTION OF THE FIGURES

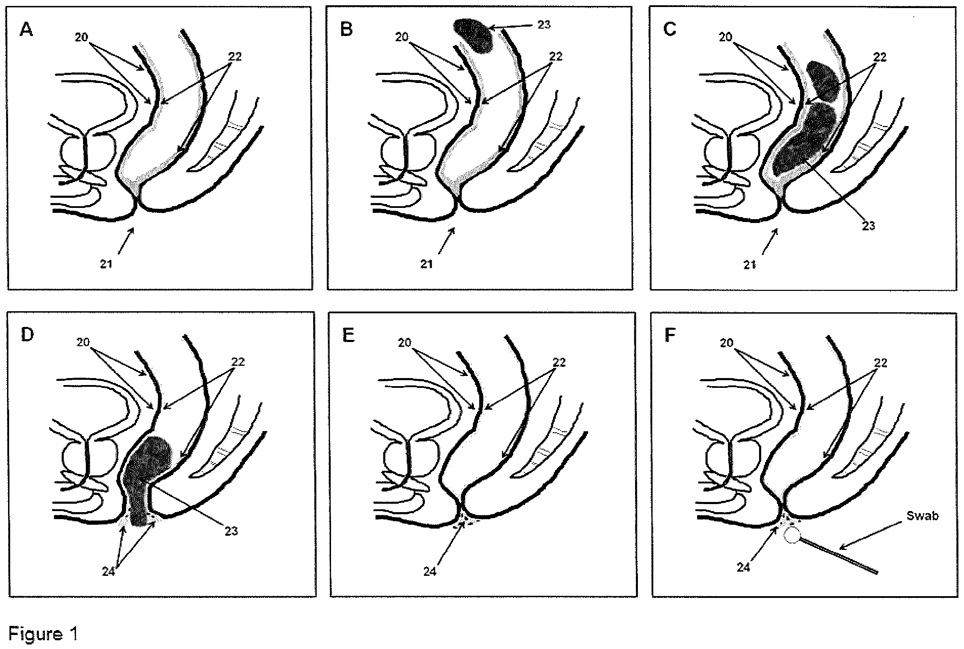

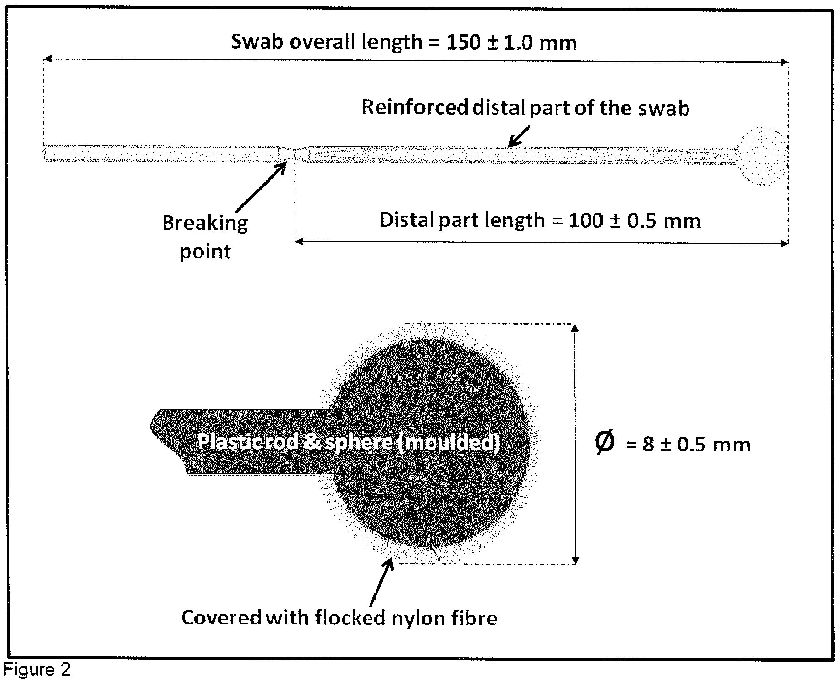

**FIGURE 1****:** shows a cross section of the human rectum, anus and adjacent organs
   A - The rectum is empty between two normal acts of defaecation. Gradual accumulation of the colonic mucocellular layer is progressing.
      20 - rectum; 21 - anus; 22 - colonic mucocellular layer overlaying the rectal mucosa.
   B - Stool (23) portions start entering the rectum. At this moment there is no significant contact between stool and the colonic mucocellular layer.
   C - The rectum is filled with stool, which comes into a close contact with the colonic mucocellular layer. Muscular contraction of the rectum preceding defaecation enhances this contact.
   D - Defaecation. Stool is being evacuated from the rectum by concerted rectal contractions and anal canal opening. A considerable proportion of the colonic mucocellular layer is evacuated together with stool, being predominantly on its surface or on the walls of the anal canal (as an additional lubricant). Fragments of the colonic mucocellular layer (24) remain on the surface of the external anal/perianal area often mixed with stool fragments.
   E - Immediately post-defaecation. The colonic mucocellular layer is considerably depleted immediately following defaecation. Its excreted fragments remain on the surface of the external anal/perianal area (and are removed by cleaning unless sample collection is planned).
   F - Post-defaecation collection of the excreted fragments of the colonic mucocellular layer using the swab described in this invention (see Figure 2).
**FIGURE 2****:** Features of the swab used for collecting colonic mucocellular layer samples from the anal area of a human subject suspected of being affected with inflammatory bowel disease immediately following bowel opening.

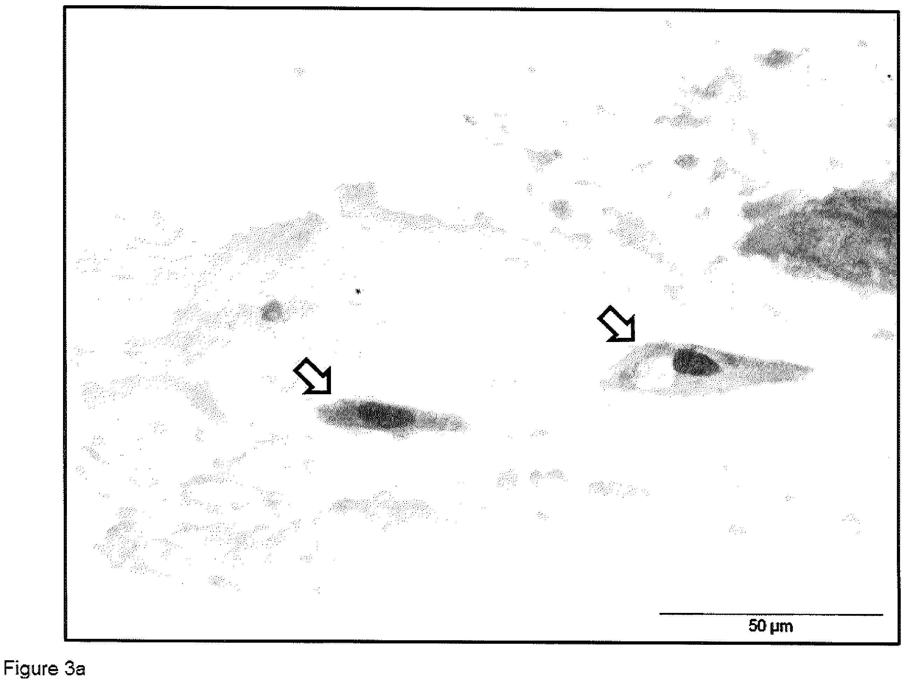

**FIGURE3a****:** Photomicrograph of a sample of colonic mucocellular layer obtained using the swab shown in Figure 2 from a healthy volunteer. The sample was placed on a microscope slide and stained with haematoxylin and eosin. The main feature of samples obtained from healthy people was scarcity of cellular material. Exfoliated normal colonocytes (two colonocytes are marked by arrows) constituted the predominant cell type in such samples.

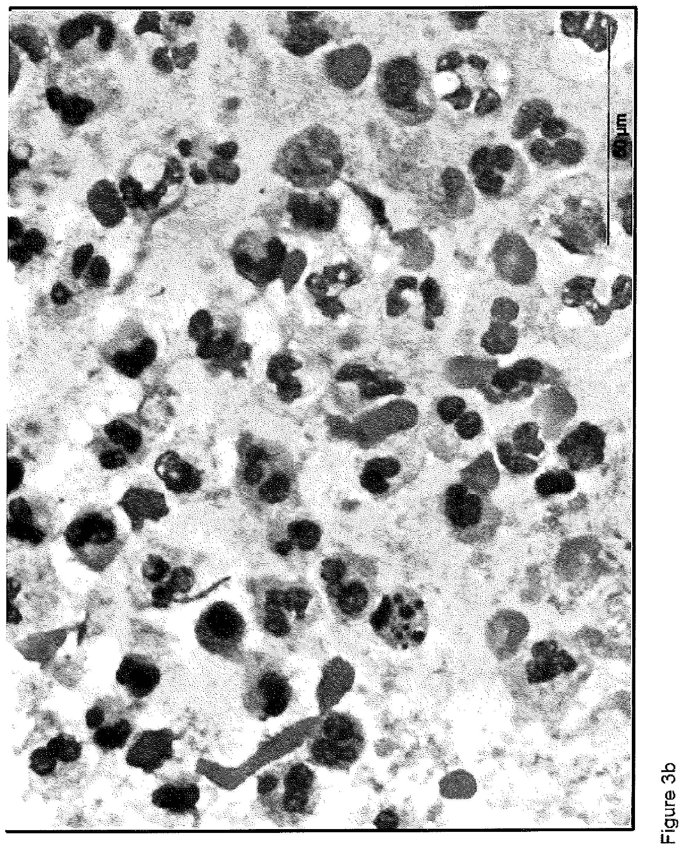

**Figure 3b****:** Photomicrograph of a sample of colonic mucocellular layer obtained using the swab shown in Figure 2 from a patient with ulcerative colitis (UC). The sample was placed on a microscope slide and stained with haematoxylin and eosin. Samples obtained from UC patients were characterised by abundant presence of very well preserved free inflammatory cells, especially polymorhonuclear leukocytes comprising neutrophils (multiple cells with segmented nuclei that can be seen) and eosinophils. Erythrocyte presence (several erythrocytes are present) was also common reflecting frequent bleeding.

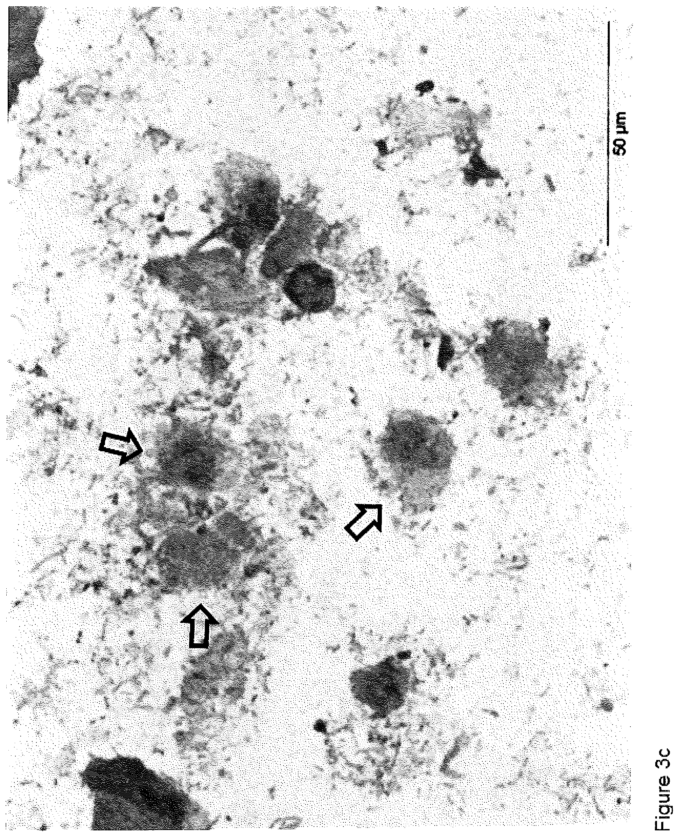

**Figure 3c****:** Photomicrograph of a sample of colonic mucocellular layer obtained using the swab shown in Figure 2 from a patient with Crohn's disease (CD) predominantly affecting ileum (proximal CD). The sample was placed on a microscope slide and stained with haematoxylin and eosin. Proximal CD cases were often characterised by the presence of distinguishable, but damaged polymorphonuclear leukocytes ("leukocyte shadows") marked by arrows.

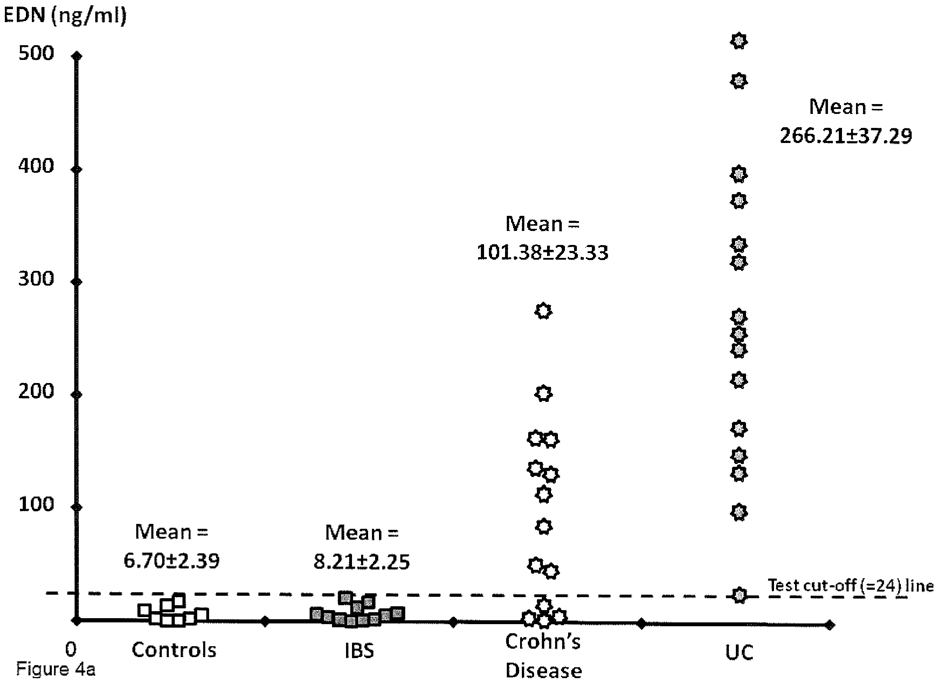

**Figure 4a****:** EDN concentrations (ng/ml) measured in lysates of colonic mucocellular layer samples obtained from eight healthy volunteers (white squares), 11 patients with IBS (grey squares), 14 patients with Crohn's disease (CD - white stars) and 15 IBD patients with UC (grey stars). All measured samples from patients with CD and UC were obtained before treatment initiation. Results in the control and IBS groups were uniformly low (all EDN concentration values were below 24 ng/ml). Among IBD patients EDN concentrations were higher in the UC subgroup, where all results were above 24 ng/ml. In the CD subgroup four results were below this threshold (all three cases of ileal CD were among them). Result distributions did not deviate from normal distribution allowing application of parametric statistics.

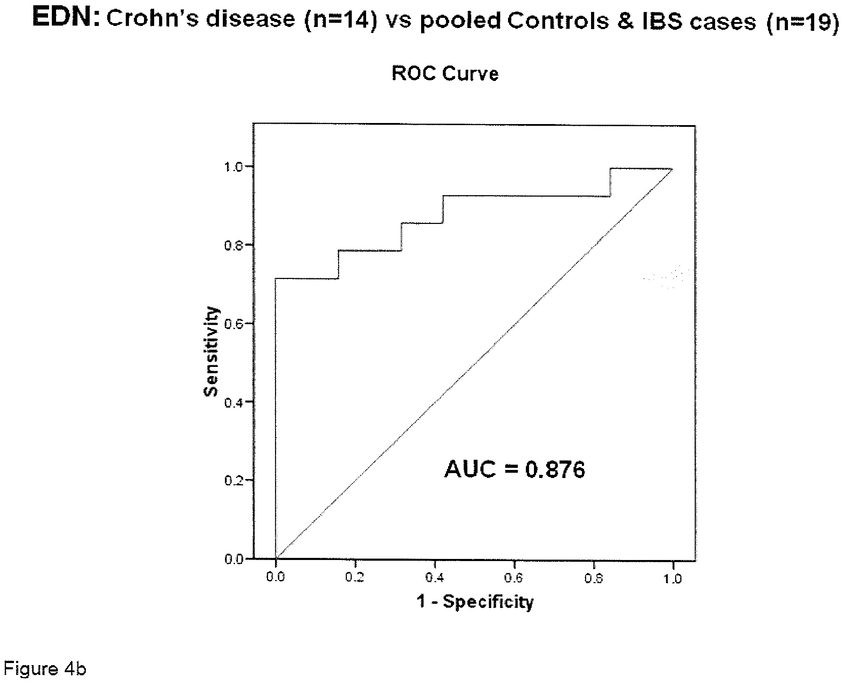

**Figure 4b****:** Receiver Operating Characteristic (ROC) curve illustrating EDN test sensitivity and specificity for detecting CD (14 patients with CD were compared with 19 individuals from control and IBS groups). Area under the curve is 0.876. At EDN concentration cut-off point = 24 ng/ml (optimal cut-off): test sensitivity = 71.4%; test specificity = 100%. At EDN concentration cut-off point = 15 ng/ml (lower limit cut-off): test sensitivity = 78.6%; test specificity = 84.2%. At EDN concentration cut-off point = 35 ng/ml (upper limit cut-off): test sensitivity = 71.4%; test specificity = 100%.

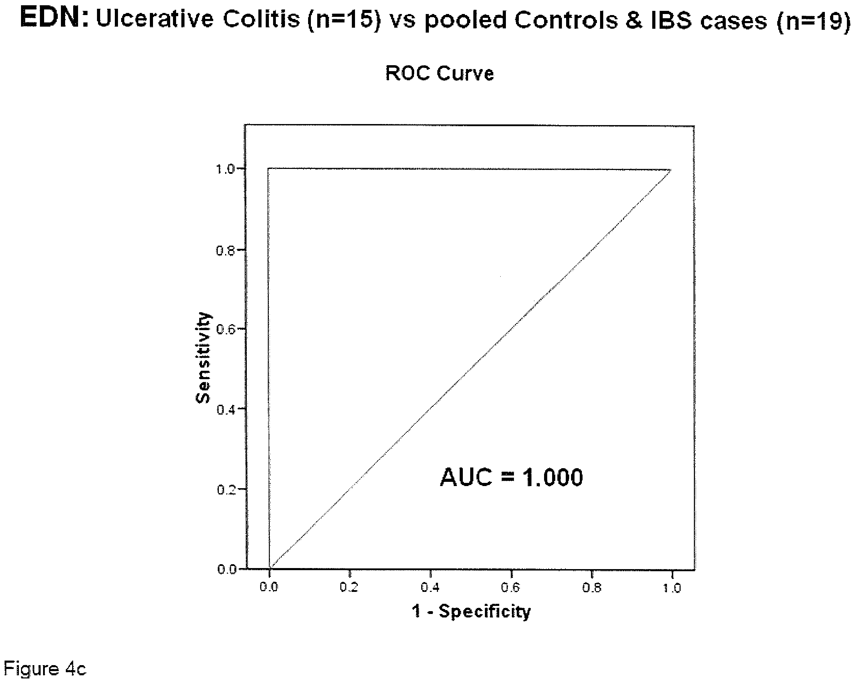

**Figure 4c****:** ROC curve illustrating EDN test sensitivity and specificity for detecting UC (15 patients with UC were compared with 19 individuals from control and IBS groups). Area under the curve is 1.000. At EDN concentration cut-off point = 24 ng/ml (optimal cut-off): test sensitivity = 100%; test specificity = 100%. At EDN concentration cut-off point = 15 ng/ml (lower limit cut-off): test sensitivity = 100%; test specificity = 84.2%. At EDN concentration cut-off point = 35 ng/ml (upper limit cut-off): test sensitivity = 93.3%; test specificity = 100%.

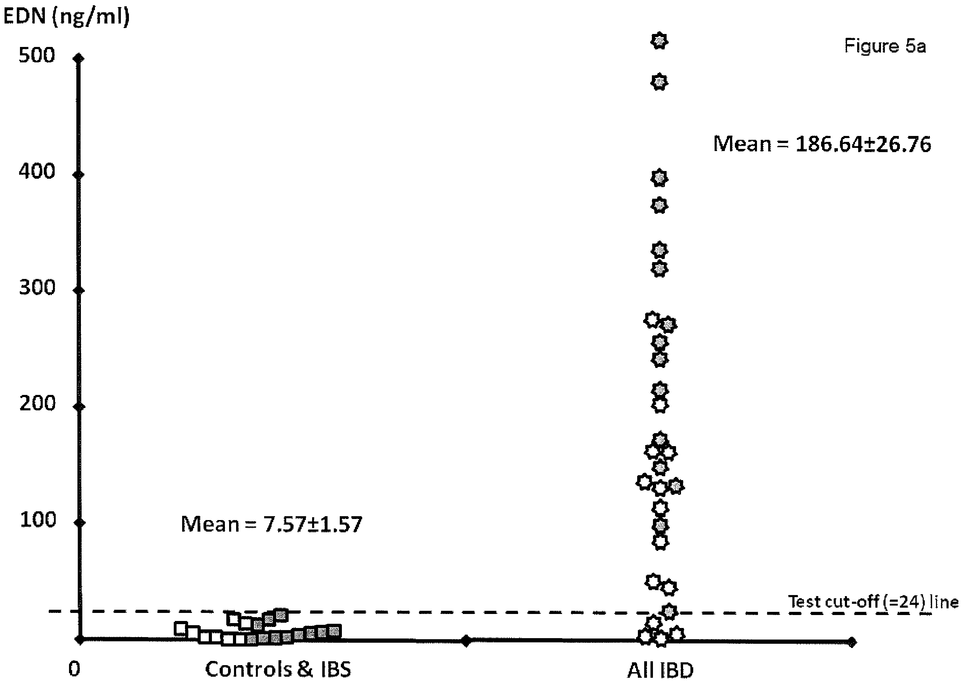

**Figure 5a****:** EDN concentrations (ng/ml) measured in lysates of colonic mucocellular layer samples obtained from healthy volunteers and patients with IBS (pooled results from 19 individuals) and IBD patients (pooled results from 29 cases). Average EDN concentration value in the IBD group was 24.7-fold higher than in the control & IBS group.

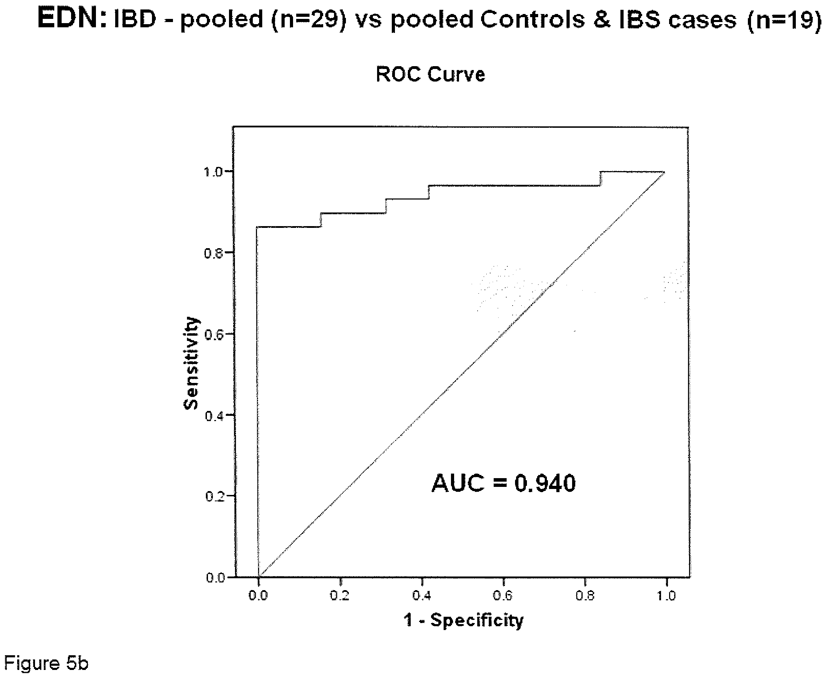

**Figure 5b****:** ROC curve illustrating EDN test sensitivity and specificity for detecting IBD (29 patients with IBD were compared with 19 individuals from control and IBS groups). Area under the curve is 0.940. At EDN concentration cut-off point = 24 ng/ml (optimal cut-off): test sensitivity = 86.2%; test specificity = 100%. At EDN concentration cut-off point = 15 ng/ml (lower limit cut-off): test sensitivity = 89.7%; test specificity = 84.2%. At EDN concentration cut-off point = 35 ng/ml (upper limit cut-off): test sensitivity = 82.8%; test specificity = 100%.

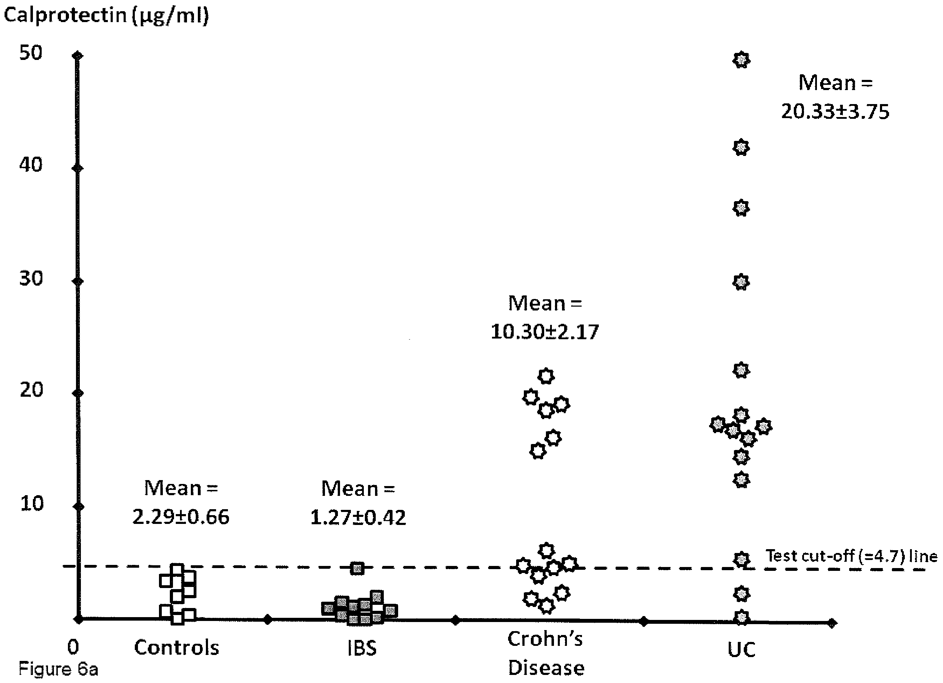

**Figure 6a****:** Calprotectin concentrations (µg/ml) measured in lysates of colonic mucocellular layer samples obtained from eight healthy volunteers (white squares), 11 patients with IBS (grey squares), 14 patients with Crohn's disease (CD - white stars) and 15 IBD patients with UC (grey stars). All measured samples from patients with CD and UC were obtained before treatment initiation. Results in the control and IBS groups were uniformly low (all calprotectin concentration values are below 4.7 µg/ml). Among IBD patients calprotectin concentrations were higher in the UC subgroup, where all results except two were above 4.7 µg/ml. In the CD subgroup four results were below this threshold (there were no ileal CD cases among them). Result distributions did not deviate from normal distribution allowing application of parametric statistics.

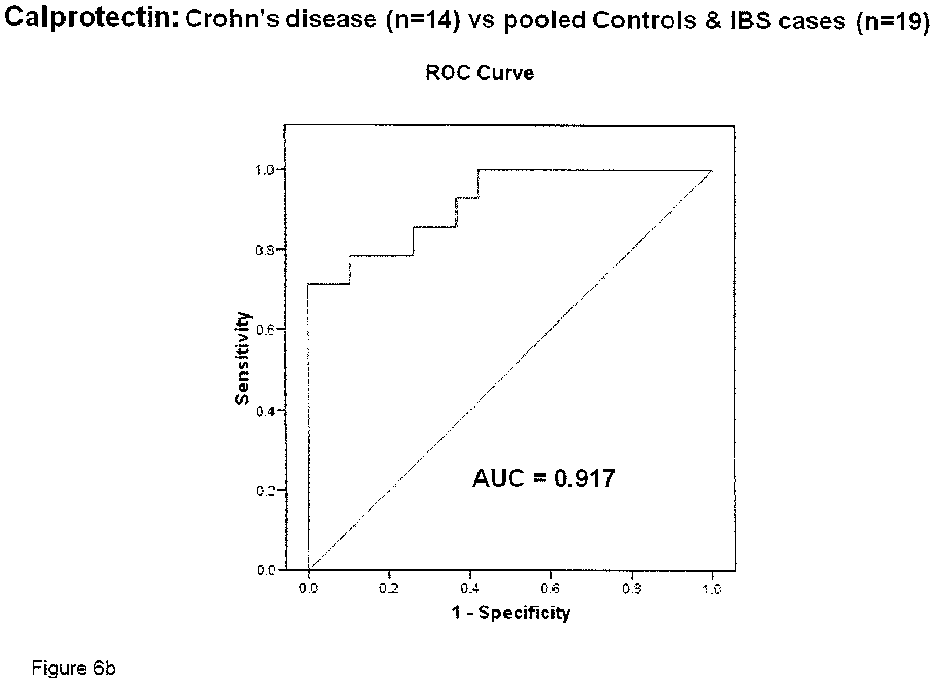

**Figure 6b****:** ROC curve illustrating calprotectin test sensitivity and specificity for detecting CD (14 patients with CD were compared with 19 individuals from control and IBS groups). Area under the curve is 0.917 (slightly higher than for EDN). At calprotectin concentration cut-off point = 4.7 µg/ml (optimal cut-off): test sensitivity = 71.4%; test specificity = 100%. At calprotectin concentration cut-off point =3.5 µg/ml (lower limit cut-off): test sensitivity = 78.6%; test specificity = 78.9%. At calprotectin concentration cut-off point = 6.0 µg/ml (upper limit cut-off): test sensitivity = 50.0%; test specificity = 100%.

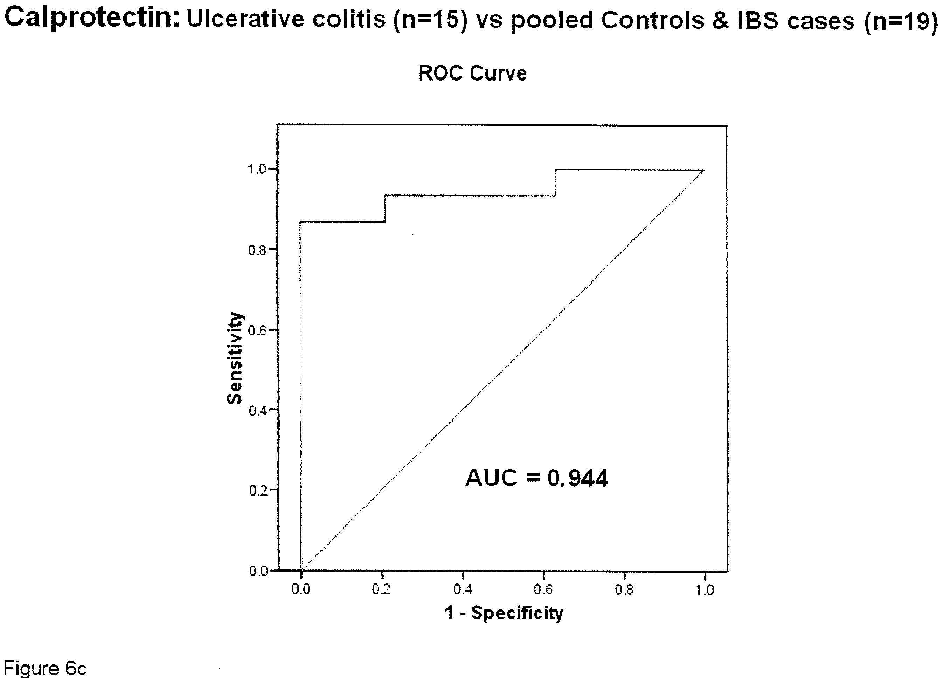

**Figure 6c****:** ROC curve illustrating calprotectin test sensitivity and specificity for detecting UC (15 patients with UC were compared with 19 individuals from control and IBS groups). Area under the curve is 0.944 (lower than for EDN). At calprotectin concentration cut-off point = 4.7 µg/ml (optimal cut-off): test sensitivity = 86.7%; test specificity = 100%. At calprotectin concentration cut-off point = 3.5 µg/ml: test sensitivity = 86.7%; test specificity = 78.9%. At calprotectin concentration cut-off point = 6.0 µg/ml (upper limit cut-off): test sensitivity = 80.0%; test specificity = 100%.

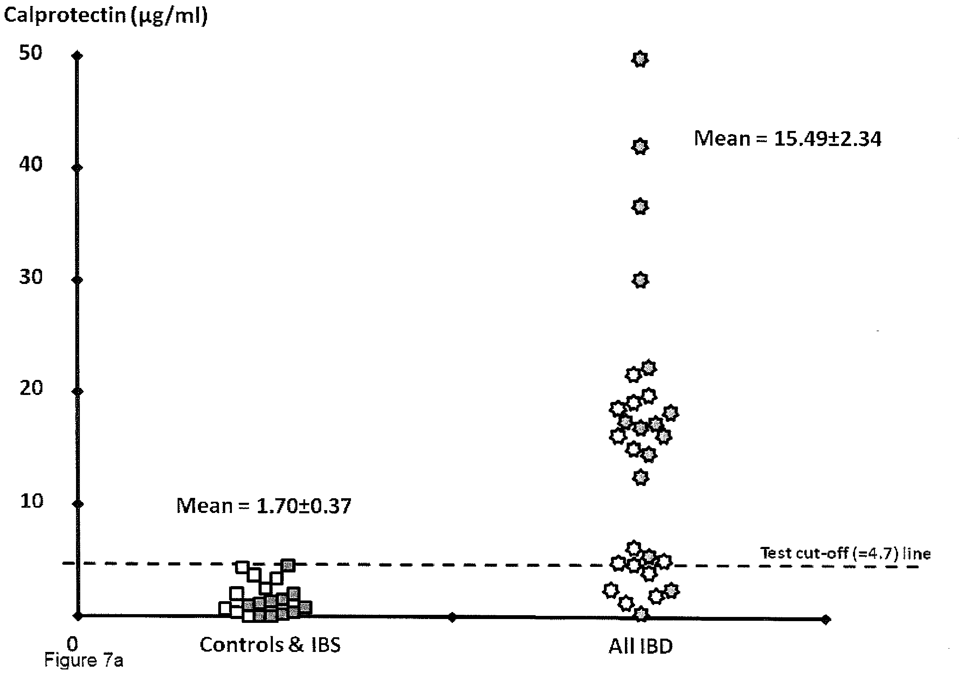

**Figure 7a****:** Calprotectin concentrations (µg/ml) measured in lysates of colonic mucocellular layer samples obtained from healthy volunteers and patients with IBS (pooled results from 19 individuals) and IBD patients (pooled results from 29 cases). Average calprotectin concentration value in the IBD group was 9.1-fold higher than in the control & IBS group.

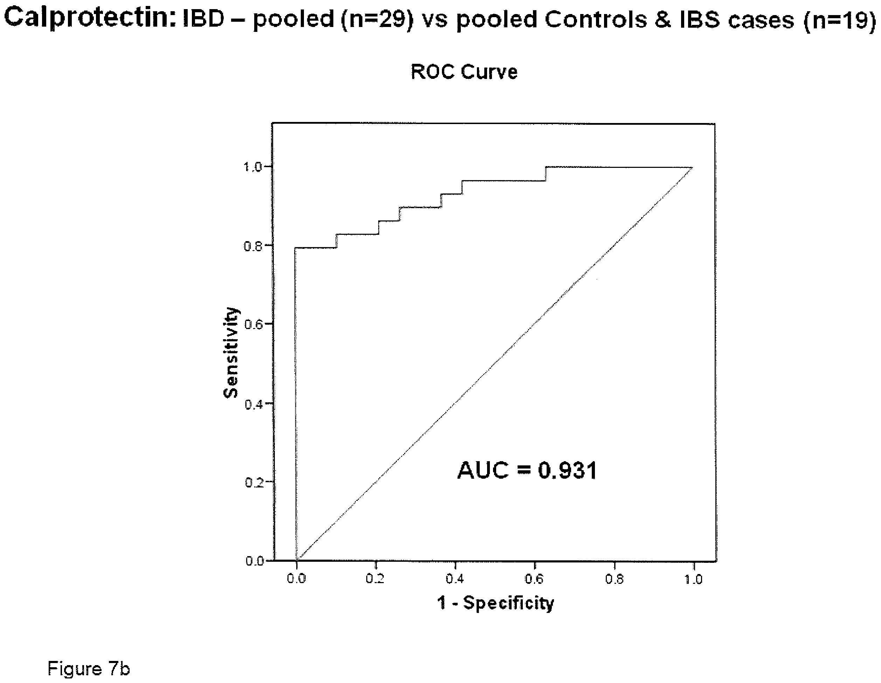

**Figure 7b****:** ROC curve illustrating calprotectin test sensitivity and specificity for detecting IBD (29 patients with IBD were compared with 19 individuals from control and IBS groups). Area under the curve is 0.931. At calprotectin concentration cut-off point = 4.7 µg/ml (optimal cut-off): test sensitivity = 79.3%; test specificity = 100%. At calprotectin concentration cut-off point = 3.5 µg/ml (lower limit cut-off): test sensitivity = 82.8%; test specificity = 78.9%. At calprotectin concentration cut-off point = 6.0 µg/ml (upper limit cut-off): test sensitivity = 65.5%; test specificity = 100%.Overall performance of the calprotectin test for IBD detection appeared to be inferior in comparison with the EDN test.

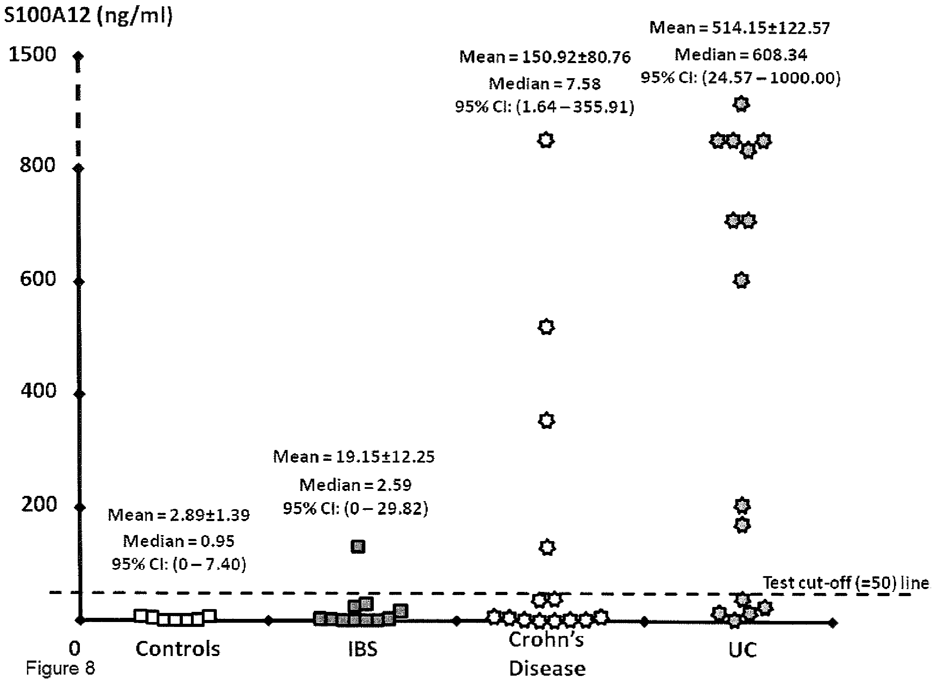

**FIGURE 8****:** S100A12 protein concentrations (ng/ml) measured in lysates of colonic mucocellular layer samples obtained from seven healthy volunteers (white squares), 11 patients with IBS (grey squares), 14 patients with Crohn's disease (CD - white stars) and 15 IBD patients with UC (grey stars). All measured samples from patients with CD and UC were obtained before treatment initiation. Results in the control and IBS groups were mostly low. A wide range of results was observed among IBD patients. In the CD group the majority of patients had low S100A12 concentrations, whereas elevated levels of this protein were detected in most cases of UC. Application of parametric statistics produced visibly exaggerated mean estimates in the IBS and CD groups, therefore median values and 95% confidence intervals are shown as well. Although S100A12 test could be considered for UC detection, its performance for detecting CD and IBD in general was clearly inferior compared to EDN and calprotectin tests.

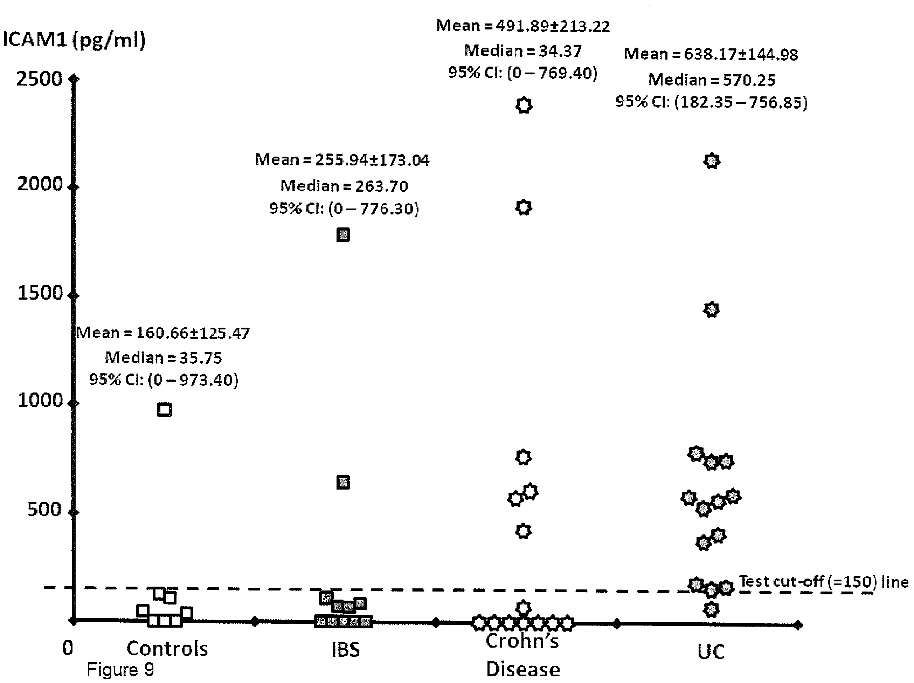

**FIGURE 9****:** ICAM1 protein concentrations (pg/ml) measured in lysates of colonic mucocellular layer samples obtained from eight healthy volunteers (white squares), 11 patients with IBS (grey squares), 14 patients with Crohn's disease (CD - white stars) and 15 IBD patients with UC (grey stars). All measured samples from patients with CD and UC were obtained before treatment initiation. Results in the control and IBS groups were mostly low, however high values were observed in one control and two IBS patients. Result distribution was clearly bimodal in the CD group with no ICAM1 presence detectable in seven cases. In contrast, ICAM1 was detected in all UC cases (only one result below 150pg/ml). Application of parametric statistics produced clearly exaggerated mean estimates in all groups except UC, therefore median values and 95% confidence intervals are shown as well. The results showed that ICAM1 test was not a good candidate for IBD detection, but allowed to identify an important subset of patients with CD.

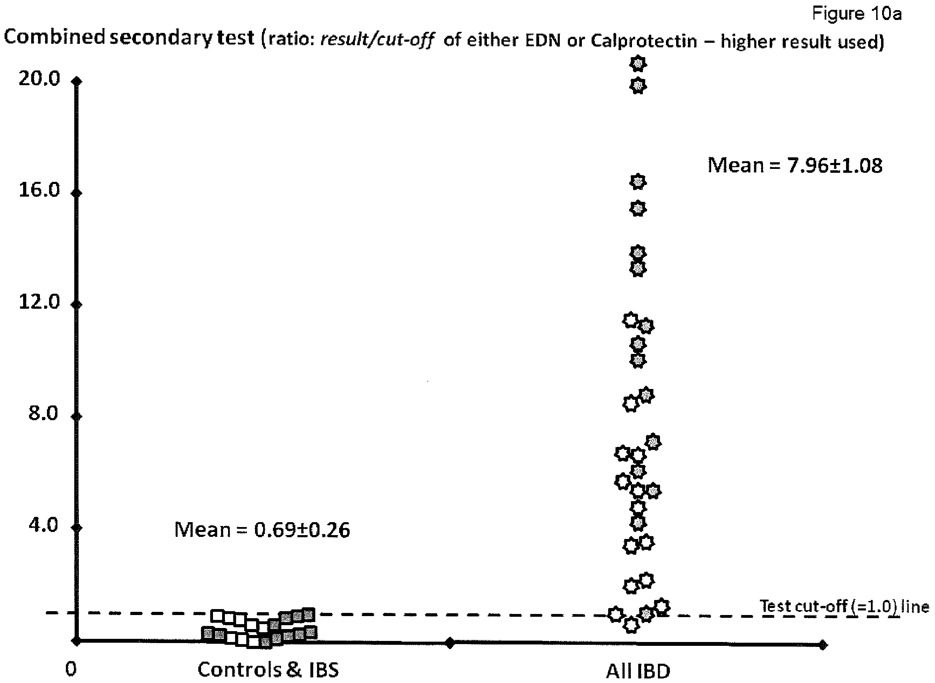

**Figure 10a****:** Results of combined secondary analysis (combined test) based upon the use of EDN and calprotectin measurement in lysates of colonic mucocellular layer samples obtained from healthy volunteers and patients with IBS (pooled results from 19 individuals) and IBD patients (pooled results from 29 cases). Two-step analysis was applied. The first step comprised determination of the following two ratios: a) detected EDN concentration/EDN test optimal cut-off point (24ng/ml); b) detected calprotectin concentration/calprotectin test optimal cut-off point (4.7 µg/ml). At the second step the obtained two ratios for each individual were compared and the higher of each two values was used as the result for the individual. Ratios below or equal to 1.0 were interpreted as negative results, whereas ratios above 1.0 were interpreted as positive results. It is remarkable that negative results were obtained in all subjects from the control and IBS groups. In contrast, only one negative result (one CD case) was observed in the IBD group.

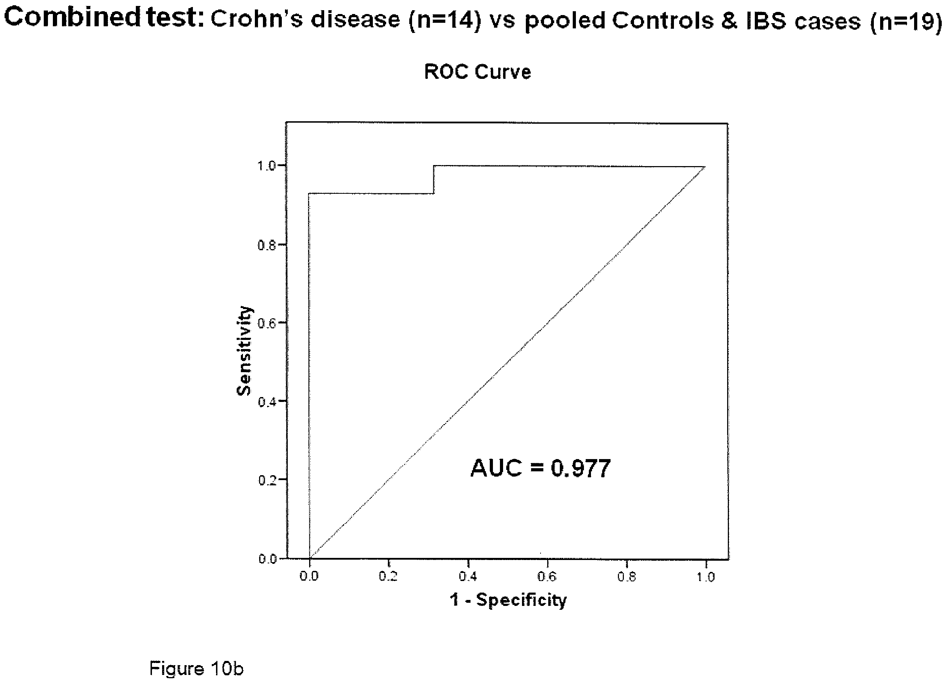

**Figure 10b****:** ROC curve illustrating combined test (see Figure 10a) sensitivity and specificity for detecting CD (14 patients with CD were compared with 19 individuals from control and IBS groups). Area under the curve is 0.977. At combined test cut-off point = 1.0: test sensitivity = 92.9%; test specificity = 100%. Combined test performance was superior compared to either EDN or calprotectin test.

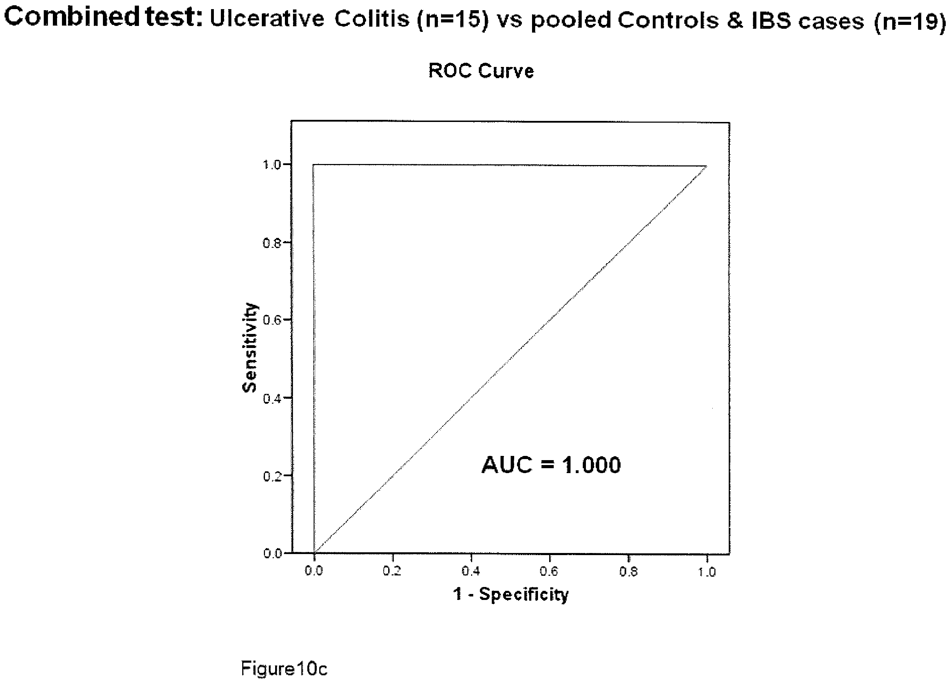

**Figure 10c****:** ROC curve illustrating combined test (see Figure 10a) sensitivity and specificity for detecting UC (15 patients with UC were compared with 19 individuals from control and IBS groups). Area under the curve is 1.00. At combined test cut-off point = 1.0: test sensitivity = 100%; test specificity = 100%. The combined test was equal to EDN test in correctly identifying all UC cases or inflammation-free subjects.

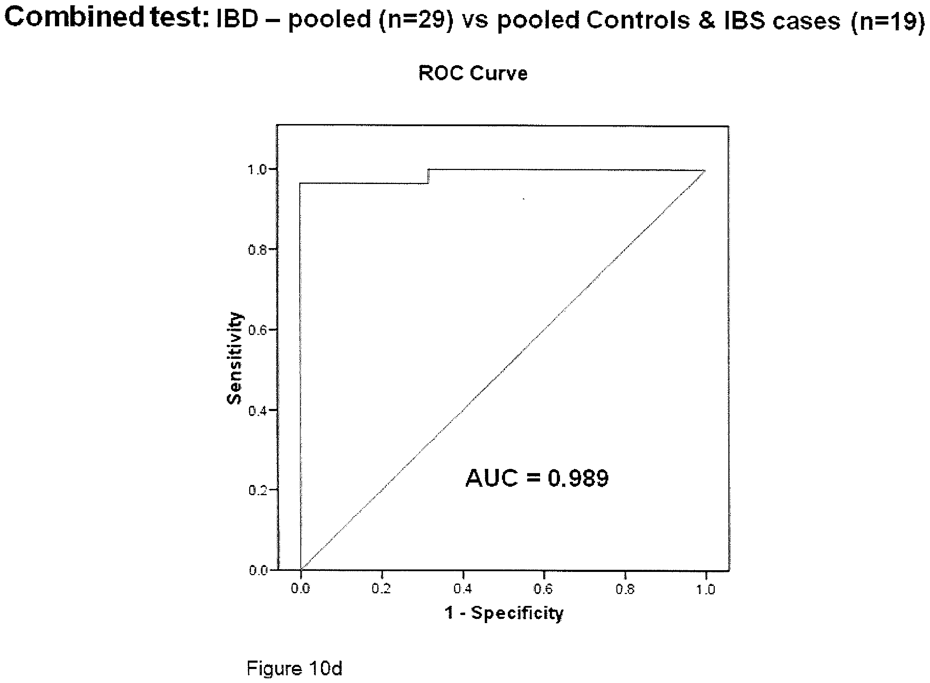

**Figure 10d****:** ROC curve illustrating combined test (see Figure 10a) sensitivity and specificity for detecting IBD (29 patients with IBD were compared with 19 individuals from control and IBS groups). Area under the curve is 0.989. At combined test cut-off point = 1.0: test sensitivity = 96.6%; test specificity = 100%. Combined test performance was superior compared to either EDN or calprotectin test due to better detection of CD cases.

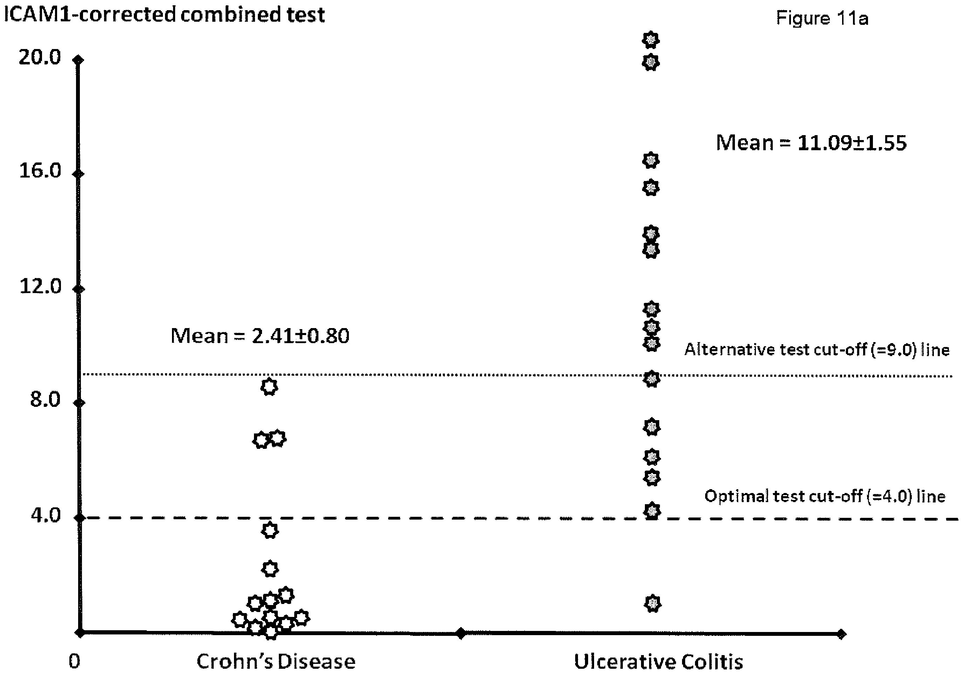

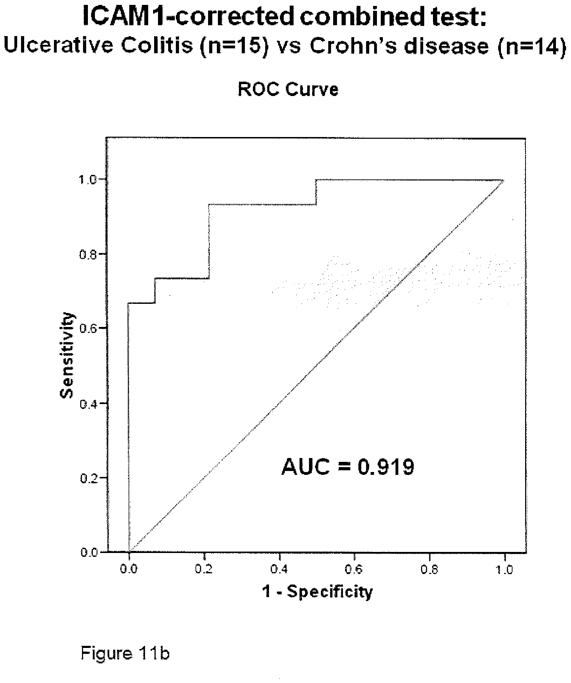

**FIGURE 11a****:** Application of the combined test with ICAM1 correction for discriminating UC from CD among IBD patients. Given that the absence of detectable ICAM1 was a characteristic feature of some CD patients, the results of the combined test (see Figure 9) were modified by introducing a multiplier of 0.1 only in the cases where no ICAM1 could be detected in the corresponding sample. In all other cases the results were not changed. The figure shows ICAM1-corrected combined test results in 14 CD cases (white stars) and 15 UC cases (grey stars) demonstrating a significant difference between the two groups. The optimal cut-off point for the combined test (=4.0) providing the best combination of sensitivity and specificity obtained by the analysis of the corresponding ROC curve (see Figure 11b). An alternative cut-off point = 9.0 providing UC detection with 100% specificity is also shown.
**Figure 11b****:** ROC curve illustrating sensitivity and specificity of ICAM1-corrected combined test (see Figure 11a) for discriminating UC from CD among IBD patients (14 patients with CD were compared with 15 patients with UC). Area under the curve is 0.919. At ICAM1-corrected combined test cut-off point = 4.0: test sensitivity = 93.3%; test specificity = 78.6%. At ICAM1-corrected combined test alternative cut-off point = 9.0 (targeting high test specificity): test sensitivity = 60.0%; test specificity = 100%.

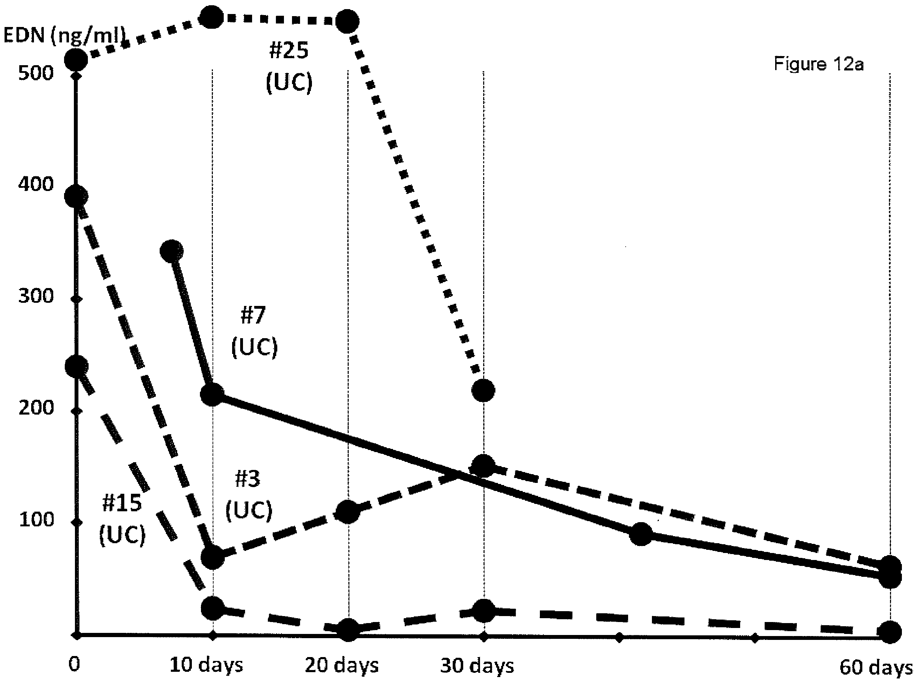

**Figure 12a****:** Dynamics of changes in EDN concentrations (ng/ml) in lysates of colonic mucocellular layer samples obtained from four IBD patients (all UC cases) before therapy initiation and at different time points during therapy. EDN concentration changes reflect changes in colonic inflammation intensity in these patients. Considerable decrease in EDN concentrations is observed in all four patients by day 30 following treatment initiation indicates reduction of inflammation intensity resulting from successful therapeutic interventions.

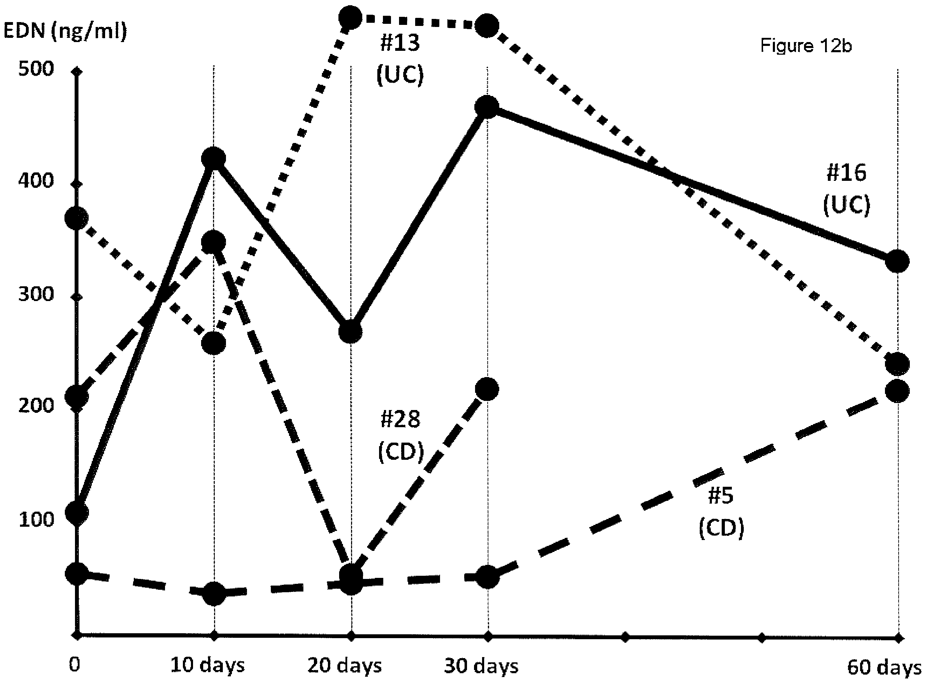

**Figure 12b****:** Dynamics of changes in EDN concentrations (ng/ml) in lysates of colonic mucocellular layer samples obtained from other four IBD patients (two UC and two CD cases) before therapy initiation and at different time points during therapy. Increasing or disorderly fluctuating EDN concentrations suggest that therapeutic interventions may require modifications in order to provide adequate anti-inflammatory effect in these patients.

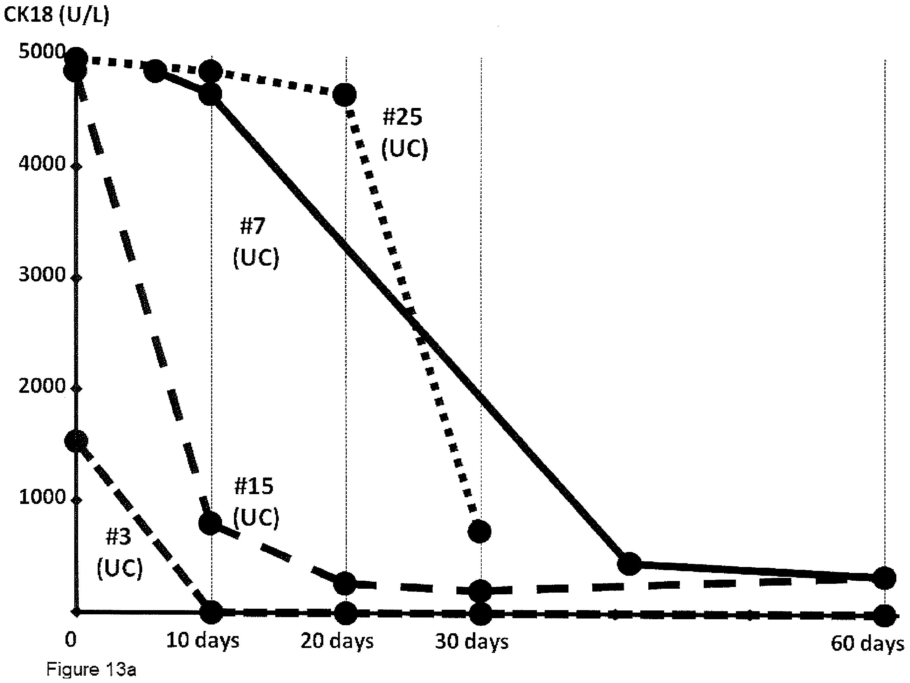

**Figure 13a****:** Dynamics of changes in soluble cytokeratin - 18 (CK18) concentrations (U/I) measured by M65 assay in lysates of colonic mucocellular layer samples obtained from four IBD patients (all UC cases already shown in Figure 12a) before therapy initiation and at different time points during therapy. CK18 patterns are likely to reflect the presence of epithelial damage in the colonic mucosa of these patients. Considerable decrease in CK18 concentrations is observed in all four patients by day 30 following treatment initiation indicating positive therapeutic effect manifested by mucosal healing.

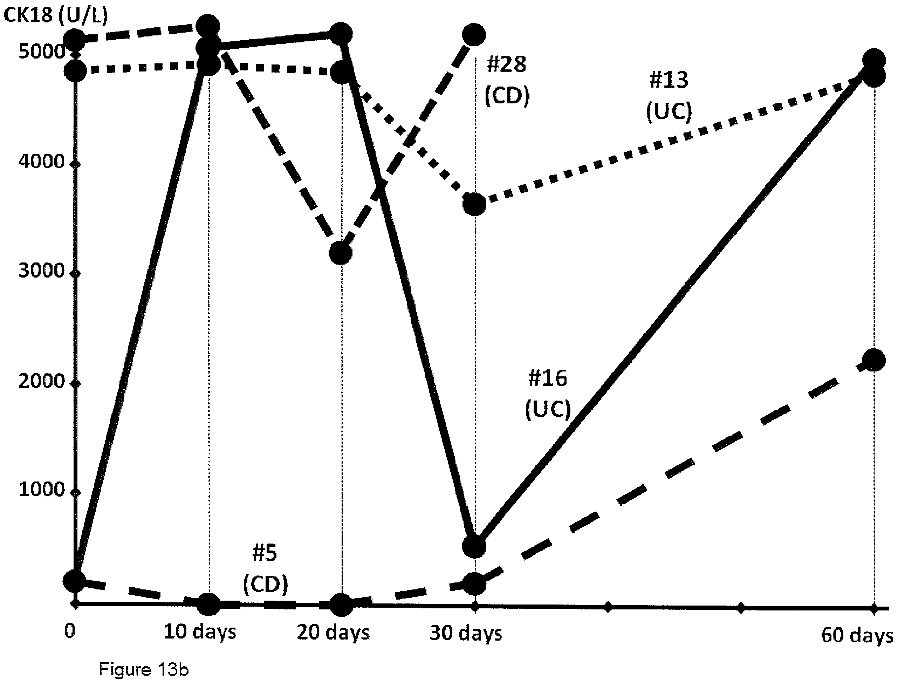

**Figure 13b****:** Dynamics of changes in soluble cytokeratin - 18 (CK18) concentrations (U/I) measured by M65 assay in lysates of colonic mucocellular layer samples obtained from other four IBD patients (two UC and two CD cases already shown in Figure 12b) before therapy initiation and at different time points during therapy. Persistently high or disorderly fluctuating CK18 concentrations suggest that therapeutic interventions may require modifications in order to provide adequate epithelial healing in these patients.

### DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features being indicated as being preferred or advantageous.

As previously discussed there exists a need to develop new methods for the diagnosis of inflammatory bowel diseases. In particular, there exists a particular need to develop a non-invasive method for the detection of these diseases.

The inventors have surprisingly identified that a considerable portion of the colonic mucocellular layer is evacuated together with stool, and more importantly fragments of the mucocellular layer remain on the surface of the external anal/perianal area following defaectaion. Usually these fragments would be removed in the course of cleaning following defaecation. However, it has been identified that these fragments can be successfully collected and subsequently analysed. The present inventors have also surprisingly identified that within this sample a number of biomarkers, specifically IBD-specific biomarkers, can be accurately detected and their concentration levels measured, and moreover, that there exists a threshold value for these biomarkers wherein concentration values above this threshold is indicative of IBD.

Moreover, the inventors have identified that the mechanism of defaecation provides a level of physiological standardisation in the sample that can subsequently be obtained as a result from the surface of the external anal/perianal area. Such a level of uniformity in the sample cannot be obtained if the sample is obtained using an invasive procedure, such as using a swab to directly sample the rectal mucosa (internally) or by using previously devices for the collection of such a sample.

The physiological mechanisms behind this method of sample collection are presented in Fig 1. It is evident that the existence and role of the mucocellular layer in the human large bowel is presently largely overlooked with only a few relevant publications (Loktionov, 2007; Loktionov et al., 2009; Loktionov et al., 2010).

Fig 1A & 1B shows that when the rectum is empty between two acts of defaecation the colonic mucocellular layer is not affected by the presence of faeces, which was demonstrated by its intrarectal collection (Loktionov et al., 2009; Loktionov et al., 2010). Once the rectum is filled with stool (see Fig 1C), the mucocellular layer comes into a close contact with stool, which is further enhanced by muscular contraction of the rectum preceding defaecation. This results in partial attachment of the mucocellular layer to excreted stool. This part of the mucocellular layer is then evacuated together with stool (Fig 1D). The phenomenon is reflected by the detectable presence of mucocellular layer elements on the surface of excreted stool (Loktionov et al., 1998; Bandaletova et al, 2002; US Pat. No. 6,187,546). At the same time it has been found that the position of the mucocellular layer between the intestinal wall and excreted faeces leaves a considerable part of the cell-containing mucus on the surface of the anal canal and eventually on the surface of the anal and perianal area (see Fig 1D & 1E). Although faecal contamination of various degrees is also present in this area following defaecation, the presence of colon-derived cellular material is significant. These conditions create an excellent opportunity for non-invasively collecting mucocellular layer fragments from the anal/perianal area as shown in Fig 1F. Collection of mucocellular layer fragments from this site has never been attempted before.

It should also be noted that the colonic mucocellular layer should be depleted in the rectum following defaecation. For this reason the necessity of the distinct physiological act of defaecation as a prerequisite for sample collection according to the method disclosed in this invention constitutes an important natural standardizing factor. Furthermore, the necessity of collecting samples immediately after defaecation makes sample self-collection the preferable way of using the device. It should also be stressed that absolute non-invasiveness of the sample collection method makes repeated sample collection extremely easy (it can be repeated following the next defaecation without any harm).

Once a sample of the colonic mucocellular layer is collected, it is placed in a tube with a buffer or medium. Then the sample can be either shipped to a laboratory for analytical assessment or immediately analysed using a lateral flow assay or a biosensor-based quantitative test.

By 'inflammatory bowel disease' or 'IBD' is meant a group of chronic disorders involving bowel inflammation. Included within this group are Crohn's disease (including ileitis, Ileocolic, and colitis) and ulcerative colitis (including distal, proctitis, proctosigmoiditis, left-sided colitis, extensive colitis and pancolitis), Inflammatory bowel disease may also include microscopic colitis (comprising collagenous colitis and lymphocytic colitis), ischaemic colitis, diversion colitis, allergic colitis, indeterminate colitis and Behçet's disease.

The term 'eosinophil-derived neurotoxin' or 'EDN' refers to the protein encoded by the RNASE2 gene and can be represented by the following sequence (SEQ ID NO: 1):

SEQ ID NO: 1:

mvpklftsqi clllllglla vegslhvkpp qftwaqwfet qhinmtsqqc tnamqvinny
qrrcknqntf llttfanvvn vcgnpnmtcp snktrknchh sgsqvplihc nlttpspqni
sncryaqtpa nmfyivacdn rdqrrdppqy pvvpvhldri i

The nucleic acid sequence encoding EDN is M30510.1.

The term 'calprotectin' refers to different heterodimers and tetramers of the proteins encoded by the S100 calcium-binding protein A8 (S100A8) gene and by the S100 calcium-binding protein A9 (S100A9) and can be represented by the following sequences (SEQ ID No: 2 and SEQ ID No: 3):

SEQ ID NO: 2:

mltelekaln siidvyhkys likgnfhavy rddlkkllet ecpqyirkkg advwfkeldi
ntdgavnfqe flilvikmgv aahkkshees hke

The nucleic acid sequence encoding S1008A is NM_002964.4.

SEQ ID NO: 3

mtckmsqler nietiintfh qysvklghpd tlnqgefkel vrkdlqnflk kenknekvie
himedldtna dkqlsfeefi mlmarltwas hekmhegdeg pghhhkpglg egtp

The nucleic acid sequence encoding S100A9 is NM_002965.3.

The term 'S100A12' refers to the protein encoded by the S100A12 gene and can be represented by the following sequence (SEQ ID NO: 4).

SEQ ID NO: 4:

mtkleehleg ivnifhqysv rkghfdtlsk gelkqlltke lantiknikd kavideifqg
ldanqdeqvd fqefislvai alkaahyhth ke

The nucleic acid encoding S100A12 is NM_005621.1.

The term 'ICAM1' or 'intracellular adhesion molecule 1' refers to the protein encoded by the ICAM1 gene and can be represented by the following sequence (SEQ ID NO: 5):

SEQ ID NO :5

mapssprpal pallvllgal fpgpgnaqts vspskvilpr ggsvlvtcst scdqpkllgi
etplpkkell lpgnnrkvye lsnvqedsqp mcysncpdgq staktfltvy wtpervelap
lpswqpvgkn ltlrcqvegg apranltvvl lrgekelkre pavgepaevt ttvlvrrdhh
ganfscrtel dlrpqglelf entsapyqlq tfvlpatppq lvsprvlevd tqgtvvcsld
glfpvseaqv hlalgdqrln ptvtygndsf sakasvsvta edegtqrltc avilgnqsqe
tlqtvtiysf papnviltkp evsegtevtv kceahprakv tlngvpaqpl gpraqlllka
tpedngrsfs csatlevagq lihknqtrel rvlygprlde rdcpgnwtwp ensqqtpmcq
awgnplpelk clkdgtfplp igesvtvtrd legtylcrar stqgevtrkv tvnvlsprye
iviitvvaaa vimgtaglst ylynrqrkik kyrlqqaqkg tpmkpntqat pp

The nucleic acid enclosing ICAM1 is NM_000201.2.

All references to the protein also encompass all alternatively spliced isoforms.

The term 'fragment' refers to a portion of the amino acid or nucleotide sequence that is less than the complete length and includes at least a minimum length capable of maintaining the biological properties required in the present invention.

The term 'variant' refers to the protein sequence where the amino acids are substantially identical to SEQ ID NO: 1, 2, 3, 4 or 5. A variant retains the biological function and activity of the protein in question. The variant may be achieved by modifications such as insertion, substitution or deletion of one or more of the amino acids. In a preferred embodiment, the variant thereof has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99% identity to SEQ ID NO:1, 2, 3 or 4.

An 'IBD-specific biomarker' refers to a protein (or nucleic acid) that causes or is associated with the presence of inflammatory bowel disease. Specifically, the biomarker may selected from the group consisting of eosinophil-derived neurotoxin (EDN), calprotectin, S100A12, ICAM1, CK-18, D-dimer, TNF-α, ASCA, pANCA, anti-GP2 antibodies, lactoferrin and total amount of human DNA in the sample. The biomarker may also be a cytokine.

A 'subject' as described herein can be any subject having an inflammatory bowel disorder. For example, the subject can be any mammal, such as a human, including a human IBD patient. Exemplary nonhuman mammals include a nonhuman primate (such as a monkey or ape) and a rodent, such as mouse or rat.

A 'lysis buffer' refers to any solution or buffer solution used for the purpose of lysing cells. The composition of the lysis buffer would be known to the skilled person. In one embodiment, the lysis buffer may comprise Tris-HCL, NaCl, Triton X-100. The lysis buffer may alternatively or additionally comprise EDTA, EGTA, SDS, deoxycholate, and/or NP-40.

The term 'treatment' refers to the management of a patient through medical or surgical means. The treatment improves or alleviates at least one symptom of a medical condition or disease and is not required to provide a cure. The treatment can encompass any known treatment for IBD. Examples include anti-inflammatory drugs. For example, the anti-inflammatory drug may be sulfasalazine, mesalamine, balsalazide, blsalazin and corticosteroids. Alternatively, the treatment may be an immune system suppressor. Examples include azathioprine, mercaptopurine, cyclosporine, infliximab, adalimumab, certolizumab pegol, methotrexate and natalizumab. Other treatments can include antibiotics (for example, metronidazole and ciprofloxacin), ICAM1 inhibitors (alicaforsen), anti-diarrheal drugs, laxatives, iron supplements, vitamin B12, calcium and vitamin D supplements or a patient-specific diet. Alternatively, the treatment may comprise surgery to remove part or all of the patient's intestine, colon and/or rectum.

The term 'antibody' as used herein refers to any immunolglobulin, preferably a full-length immunoglobulin. Preferably, the term covers monoclonal antibodies, polyclonal antibodies, multispecific antibodies, such as bispecific antibodies, intracellular antibodies (or intrabodies) and antibody fragments thereof, so long as they exhibit the desired biological activity. Antibodies may be derived from any species, but preferably are of rodent, for examples rat or mouse, human or rabbit origin. Alternatively, the antibodies, preferably monoclonal antibodies, may be humanised, chimeric or antibody fragments thereof. The term 'chimeric antibodies' may also include "primatised" antibodies comprising variable domain antigen-binding sequences derived from a non-human primate (e.g., Old World Monkey, Ape etc) and human constant region sequences. The immunoglobulins can also be of any type (e.g. IgG, IgE, IgM, IgD, and IgA), class (e.g., IgGI, IgG2, IgG3, IgG4, IgAI and IgA2) or subclass of immunoglobulin molecule.

The term 'monoclonal antibody' refers to a substantially homogenous population of antibody molecules (i.e. the individual antibodies comprising the population are identical except for possible naturally occurring mutations that may be present in minor amounts), produced by a single clone of B lineage cells, often a hybridoma. Importantly, each monoclonal has the same antigenic specificity - i.e. it is directed against a single determinant on the antigen.

The production of monoclonal antibodies can be carried out by methods known in the art. However, as an example, the monoclonal antibodies can be made by the hybridoma method (Kohler & Milstein, 1975), the human B cell hybridoma technique (Kozbor & Roder , 1983), or the EBV-hybridoma technique (Cole et al, 1985 . Alternatively, the monoclonal antibody can be produced using recombinant DNA methods (see, US Pat. App. No. 4,816,567) or isolated from phage antibody libraries using the techniques described in Clackson et al (1991) and Marks et al (1991).

Polyclonal antibodies are antibodies directed against different determinants (epitopes). This heterogenous population of antibody can be derived from the sera of immunised animals using various procedures well known in the art.

The term 'bispecific antibody' refers to an artificial antibody composed of two different monoclonal antibodies. They can be designed to bind either to two adjacent epitopes on a single antigen, thereby increasing both avidity and specificity, or bind two different antigens for numerous applications, but particularly for recruitment of cytotoxic T- and natural killer (NK) cells or retargeting of toxins, radionuclides or cytotoxic drugs for IBD treatment (Holliger & Hudson, 2005). The bispecific antibody may have a hybrid immunoglobulin heavy chain with a first binding specificity in one arm, and a hybrid immunoglobulin heavy chain-light chain pair (providing a second binding specificity) in the other arm. This asymmetric structure facilitates the separation of the desired bispecific compound from unwanted immunoglobulin chain combinations, as the presence of an immunoglobulin light chain in only one half of the bispecific molecule provides for a facile way of separation (WO94/04690; Suresh et al, 1986; Rodrigues et al, 1993; ; Carter et al, 1992; Carter et al, 1995; Merchant et al, 1998).

Methods to prepare hybrid or bispecific antibodies are known in the art. In one method, bispecific antibodies can be produced by fusion of two hybridomas into a single 'quadroma' by chemical cross-linking or genetic fusion of two different Fab or scFv modules (Holliger & Hudson, 2005).

The term 'chimeric' antibody refers to an antibody in which different portions are derived from different animal species. For example, a chimeric antibody may derive the variable region from a mouse and the constant region from a human. In contrast, a 'humanised antibody' comes predominantly from a human, even though it contains non-human portions. Specifically, humanised antibodies are human immunoglobulins (recipient antibody) in which residues from a hypervariable region of the recipient are replaced by residues from hypervariable regions of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanised antibodies may comprise residues that are not found in the recipient antibody or in the donor antibody. These modifications are made to further refine antibody performance. In general, the humanised antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanised antibody optionally also will comprise at least a portion of an immunoglobulin constant region (Fc), typically that of a human immunoglobulin.

Recombinant antibodies such as chimeric and humanised monoclonal antibodies can be produced by recombinant DNA techniques known in the art. Completely human antibodies can be produced using transgenic mice that are incapable of expressing endogenous immunoglobulin heavy and light chains genes, but which can express human heavy and light chain genes. The transgenic mice are immunized in the normal fashion with a selected antigen. Monoclonal antibodies directed against the antigen can be obtained using conventional hybridoma technology. The human immunoglobulin transgenes harboured by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995). For a detailed discussion of this technology for producing human antibodies and human monoclonal antibodies and protocols for producing such antibodies, see, for example, US Pat. Nos. 5,625,126; 5,633,425; 5,569,825; 5,661,016; 5,545,806. Other human antibodies can be obtained commercially from, for example, Abgenix, Inc. (Freemont, CA) and Genpharm (San Jose, CA).

The term 'antigen-binding fragment' in the context of the present invention refers to a portion of a full length antibody where such antigen-binding fragments of antibodies retain the antigen-binding function of a corresponding full-length antibody. The antigen-binding fragment may comprise a portion of a variable region of an antibody, said portion comprising at least one, two, preferably three CDRs selected from CDR1, CDR2 and CDR3. The antigen-binding fragment may also comprise a portion of an immunoglobulin light and heavy chain. Examples of antibody fragments include Fab, Fab', F(ab')2, scFv, di-scFv, and BiTE (Bi-specific T-cell engagers), Fv fragments including nanobodies, diabodies, diabody-Fc fusions, triabodies and, tetrabodies; minibodies; linear antibodies; fragments produced by a Fab expression library, anti-idiotypic (anti-Id) antibodies, CDR (complementary determining region), and epitope-binding fragments of any of the above that immunospecifically bind to a target antigen. For comparison, a full length antibody, termed 'antibody' is one comprising a VL and VH domains, as well as complete light and heavy chain constant domains.

The antibody may also have one or more effector functions, which refer to the biological activities attributable to the Fc region (a native sequence Fc region or amino acid sequence variant Fc region engineered according to methods in the art to alter receptor binding) of an antibody. Examples of antibody effector functions include Clq binding; complement dependent cytotoxicity; Fc receptor binding; antibody-dependent cell-mediated cytotoxicity (ADCC); phagocytosis; down regulation of cell surface receptors (e.g., B cell receptor; BCR), etc.

The antibody can also be a functionally active fragment, derivative or analog of an antibody that immunospecifically binds to a target antigen. In this regard, functionally active means that the fragment, derivative or analog is able to elicit anti-idiotype antibodies that recognise the same antigen that the antibody from which the fragment, derivative or analog is derived recognised. Specifically, in an exemplary embodiment the antigenicity of the idiotype of the immunoglobulin molecule can be enhanced by deletion of framework and CDR sequences that are C-terminal to the CDR sequence that specifically recognizes the antigen. To determine which CDR sequences bind the antigen, synthetic peptides containing the CDR sequences can be used in binding assays with the antigen by any binding assay method known in the art (e.g., the BIA core assay), see, for example, Kabat (1980) and Kabat et al (1991).

The term 'antibody' may also include a fusion protein of an antibody, or a functionally active fragment thereof, for example in which the antibody is fused via a covalent bond (e.g., a peptide bond), at either the N-terminus or the C-terminus to an amino acid sequence of another protein (or portion thereof, such as at least 10, 20 or 50 amino acid portion of the protein) that is not the antibody. The antibody or fragment thereof may be covalently linked to the other protein at the N-terminus of the constant domain.

Furthermore, the antibody or antigen-binding fragments of the present invention may include analogs and derivatives of antibodies or antigen-binding fragments thereof that are either modified, such as by the covalent attachment of any type of molecule as long as such covalent attachment permits the antibody to retain its antigen binding immunospecificity. Examples of modifications include glycosylation, acetylation, pegylation, phosphorylation, amidation, derivatization by known protecting/blocking groups, proteolytic cleavage, linkage to a cellular antibody unit or other protein, etc. Any of numerous chemical modifications can be carried out by known techniques, including, but not limited to specific chemical cleavage, acetylation, formylation, metabolic synthesis in the presence of tunicamycin, etc. Additionally, the analog or derivative can contain one or more unnatural amino acids.

The antibodies or antigen-binding fragments of the present invention may also have modifications (e.g., substitutions, deletions or additions) in the Fc domain of the antibody. Specifically, the modifications may be in the Fc-hinge region and result in an increased binding for the FcRn receptor (WO97/34631).

In one aspect of the methods described herein, the concentration of at least one IBD-specific biomarker, or fragment or variant thereof, is measured in a sample of the colonic mucocellular layer and compared to a threshold value.

Alternatively the threshold value may be determined by determining the concentration of the IBD-specific biomarker in a group of one or more control subjects. Preferably this group comprises at least five control subjects. More preferably this group comprises at least ten control subjects.

Thus, in the methods described herein, the methods may further comprise the following steps:
a) obtaining a sample of the colonic mucocellular layer from at least one control subject and from the test subject;
b) measuring or determining the concentration levels of said IBD-specific biomarker (for example EDN, calprotectin, ICAM1 or S100A12) or a fragment or variant thereof in said sample from the at least one control subject and the test subject
c) comparing the observed concentration levels in a test subject to the concentration levels in at least one control subject, wherein a change, preferably an increase, in the concentration levels in the test subject indicates the presence of an IBD.

In one embodiment the at least one control subject represents a control or reference cohort and the concentration of the at least one IBD-specific biomarker is compared to the median concentration level in said control or reference cohort.

The control sample (i.e. the sample obtained from the at least one control subject) is taken from a reference cohort, that is one or more subjects of the same species (e.g., human subjects). The reference cohort is preferably healthy subjects. More preferably the subjects are believed not to have an IBD (or have inflammatory bowel syndrome (IBS), but not IBD). The individual members of a reference cohort may also share other similarities, such as similarities in stage of disease, previous treatment regimens, lifestyle (e.g., smokers or nonsmokers, overweight or underweight), or other demographics (e.g., age, genetic disposition).

Alternatively, the threshold value defines a fractile of a distribution of measured concentrations for a population of control subjects without IBD. Preferably, said fractile is a 0.9 or greater fractile. Preferably, said distribution is assumed to be a Gaussian distribution.

Alternatively, the threshold value identifies that said subject is IBD-positive with greater than a threshold value. In a further alternative, said threshold value is associated with a defined probability of determining that said subject has IBD, when said subject has IBD. Preferably, said defined probability is greater than 60%, preferably 65%, preferably 70%, preferably 75%, preferably 80%, preferably 85%, preferably 90%, preferably 95%, preferably 100%.

In an alternative embodiment, the concentration levels of a biomarker (for example, EDN, calprotectin, ICAM1 or S100A12) in a sample from a test subject can be measured and compared with a predetermined reference value. The 'predetermined reference value' or 'threshold concentration' (these terms are used interchangeably herein) can be established by skilled healthcare practitioners. For instance, the predetermined reference value can be established by measuring the levels of the biomarker in a normal population sample and correlating such levels with factors such as the incidence, severity, and/or frequency of developing IBD. Thus, a subject's concentration levels of the biomarker as compared against the concentration levels in a normal population can be indicative of whether the subject has IBD. Further, the predetermined reference value is preferably established by using the same assay technique as is used for measurement of the subject's biomarker level, to avoid any error in standardization.

In a preferred embodiment, the sample of the colonic mucocellular layer from the control subject may be obtained from the surface of the anal area following defaecation.

The invention also relates to a method for monitoring the effectiveness of a treatment for IBD. In a specific embodiment, the method comprises measuring the concentration levels of an IBD-specific biomarker over the course of a treatment period. If the concentration levels rise or stay the same compared to the levels pre-treatment or increase compared to a predetermined threshold value, the treatment can be concluded to be ineffective. Alternatively, if the concentration levels decrease compared to the levels pre-treatment or decrease compared to a predetermined threshold value, the treatment can be concluded to be effective. As a consequence the treatment may need to be altered or the dosage of the existing treatment. In this method, the test sample is from a patient who has received or is receiving treatment. Thus, in one embodiment of the method for monitoring the effectiveness of a treatment the method may further comprise the steps of (i) optionally obtaining a pre-treatment sample from a patient prior to administration of the treatment; (ii) optionally detecting or measuring the concentration of the IBD-specific biomarker in the pre-treatment sample; (iii) obtaining one or more post-treatment samples from the subject at selected time intervals; (iv) determining the concentration levels of the IBD-specific biomarker in the post-treatment sample(s); (v) comparing the concentration levels of the IBD-specific biomarker in the pre-treatment sample with the concentration levels in the post-treatment sample or samples or comparing the concentration levels in the post-treatment samples taken at different time intervals; and (vi) optionally altering the administration of the treatment to the subject accordingly.

The invention also relates to a method for monitoring disease relapse in an IBD patient in remission. In a specific embodiment, the method comprises measuring the concentration levels of an IBD-specific biomarker over specified time intervals. As IBD is a life-long condition and so there is no upper time-limit for measuring the concentration levels of an IBD-specific biomarker in an IBD patient in remission. This will be the case unless the patient undergoes a radical procedure such as colectomy. As an example of the frequency, the method could comprise measuring the concentration levels of an IBD-specific biomarker once every 12 months, preferably every 6 months, more preferably every 3 months. The patient may or may not still be receiving treatment for IBD. If the concentration levels rise over the specified time intervals, the patient can be concluded to be out of remission - i.e. the disease has relapsed. Alternatively, if the concentration levels rise to be equal or greater than a threshold value the patient can be concluded to be out of remission. As a consequence the treatment may need to be altered (or the dosage of an existing treatment altered) or treatment resumed all together. However, if the concentration levels stay the same or decrease over the specified time intervals, or if the levels decrease with respect to a threshold value, the patient can be concluded to be still in remission.

Thus, in one embodiment of the method for monitoring for disease relapse in an IBD patient in remission, the method may further comprise the steps of (i) obtaining a sample from an IBD-patient in remission at one or more time intervals, ii) comparing said concentration levels to a threshold value and determining a disease relapse when the concentration of the IBD-specific biomarker is equal to or greater than a threshold value and iii) optionally re-starting or altering the administration of treatment to the subject accordingly.

We also describe a method of selecting a treatment for an IBD patient. In a specific embodiment, the method comprises measuring the concentration levels of an IBD-specific biomarker and comparing the concentration level to a threshold value. If the concentration of the IBD-specific biomarker is above this threshold an IBD-specific treatment may be selected. However, if the concentration of the IBD-specific biomarker is below this threshold, no treatment may be selected. In one example, the concentration of the IBD-specific biomarker is indicative that the patient will respond to a specific type of therapy. This specific therapy may be directed against or be specific to the IBD-specific biomarker in question. For example, if the IBD-specific biomarker is ICAM1, the IBD-specific treatment may be alicaforsen. Alternatively, if the IBD-specific biomarker is TNF-α, the IBD-specific treatment may be infliximab. Accordingly, we also describe herein a method of tailoring or selecting a specific IBD-treatment depending on which IBD-specific biomarker is elevated in the patient's sample.

Accordingly, we further describe here, a method of predicting a response to a particular IBD-specific treatment (for example a ICAM1-targeted therapy or other IBD biomarker-targeted therapy), the method comprising measuring the concentration levels of at least one IBD-specific biomarker, comparing the concentration level(s) to a threshold value and determining whether a subject will respond to a particular IBD-specific treatment depending on whether the concentration of the IBD-specific biomarker is equal to or greater than a threshold value.

The threshold value for an IBD-specific biomarker may be any threshold value already mentioned above.

We further describe herein, a method for assessing colonic mucosa damage. In other words, we describe a method for assessing whether the colonic mucosa is healthy. Preferably, the method comprises measuring the concentration levels of at least one epithelial damage-specific biomarker, comparing the concentration level(s) to a threshold value and determining that the colonic mucosa is damaged when the concentration of at least one epithelial damage-specific biomarker is equal to or greater than a threshold value.

The term 'epithelial damage specific biomarkers' means biomolecules released during epithelial cell death or their metabolites. Examples include proteins such as soluble cytokeratine - 18 (CR-18) and intestinal fatty acid binding protein (I-FATP) and smaller molecules such as α-amino acide citrulline.

In one example the epithelial damage-specific biomarker is CK-18 (Cytokeratin-18) and the threshold value is, or is equivalent to 300 U/L when the collected sample is lysed in 3ml of lysis buffer.

In an alternative example the concentration of CK-18 is, or is equivalent to a value, equal to or greater than 500 U/L when the collected sample is lysed in 3ml of lysis buffer. In a further alternative the concentration of CK-18 is or is equivalent to a value, equal or greater than a concentration value within the range 100 U/L to 35 U/L when the collected sample is lysed in 3ml of lysis buffer.

In the methods described herein, the concentration of the IBD-specific biomarker (for example calprotectin, EDN, ICAM1 or S100A12) can be determined by measuring protein levels (i.e. protein concentration) of the respective IBD-specific biomarker. In one embodiment protein levels can be measured by contacting the sample with at least one binding agent, such as an antibody, that is capable of specifically binding the IBD-specific biomarker in question.

In the embodiments of this invention where one uses antibodies against a protein IBD-specific biomarker for diagnostic purposes, one can select any immunogenic fragment of the biomarker peptide to raise an antibody as is well known to one skilled in the art. The fragments that are immunogenic will lead to generation of antibodies. Protein fragments can be readily screened for immunogenic activity. Preferably, one uses monoclonal antibodies, but one can also use polyclonal antibodies. One can perform an immunohistochemical analysis using a polyclonal or monoclonal antibody raised against the entire biomarker peptide, or any fragments thereof.

In a further aspect the method further comprises determining whether the antibody has bound to the protein - i.e. whether an antibody-antigen complex has formed. In one aspect the formation of an antibody-antigen complex is measured using immunoassay. In one example the immunoassay is an ELISA kit. Alternatively bound antibody can be detected using any method known in the art. Examples include 2D gels and ID SDS-PAGE followed by western blotting, dot-blots, and flow cytometry. Alternatively bound antibody is measured using a lateral flow assay or in a quantitative biosensor-based assay employing electrochemical biosensors.

Accordingly, the methods further comprise detection or quantitation of the antigen-antibody complex. Such methods would be well known to the skilled person. For example, any chemical that detects antigen-antibody binding may be used in the practice of the invention. In some embodiments, the detection chemicals comprise a labelled polymer conjugated to a secondary antibody. For example, a secondary antibody that is conjugated to an enzyme that catalyses the deposition of a chromogen at the antigen-antibody binding site may be provided. Such enzymes and techniques for using them in the detection of antibody binding are well known in the art. In one embodiment the secondary antibody is conjugated to an HRP-labelled polymer. Alternatively, the antigen-antibody complex can be detected using any commercial antibody binding detection system. Examples include BIO-PLEX by Bio-Rad and fluorescent labels.

Thus, in one embodiment of the methods of the invention, the methods further comprise the following steps:
(a) contacting a test sample from a subject with a binding agent specific for the biomarker in question, e.g. an antibody as described herein, which is directly or indirectly labeled with an enzyme;
(b) adding a substrate for the enzyme wherein the substrate is selected so that the substrate, or a reaction product of the enzyme and substrate, forms fluorescent complexes;
(c) quantitating protein concentration levels in the sample by measuring fluorescence of the fluorescent complexes; and
(d) comparing the quantitated levels to that of a standard.

Another embodiment of the invention comprises the following steps:
(a) incubating a test sample with a first antibody specific for the IBD-specific biomarker in question which is directly or indirectly labeled with a detectable substance, and a second antibody specific for the biomarker which is immobilized;
(b) separating the first antibody from the second antibody to provide a first antibody phase and a second antibody phase;
(c) detecting the detectable substance in the first or second antibody phase thereby quantitating protein concentration in the biological sample; and
(d) comparing the quantitated protein levels with a control or threshold value.

Alternatively, the concentration of the IBD-specific biomarker can be determined by measuring expression levels of the respective IBD-specific biomarker. This may comprise detection or measurement of DNA or RNA of the IBD-specific biomarker. Methods to detect RNA or DNA levels in a sample are well known to the skilled person and include any nucleic-acid hybridisation-based technique or amplification-based technique such as PCR (including all forms of PCR, such as real-time),a lateral flow assay or an electrochemical biosensor-based assay.

Kits for practicing the methods of the invention are further provided. A 'kit' refers to any manufacture (e.g., a package or a container) comprising at least one reagent, e.g. a binding agent such as an antibody, for specifically detecting the concentration levels of an IBD-specific biomarker described herein. The kit may be promoted, distributed, or sold as a unit for performing the methods of the present invention. Additionally, the kits may contain a package insert describing the kit and methods for its use. The kit may also comprise a solid support such as microtiter multi-well plates, a lateral flow assay or an electrochemical biosensor-based assay.

For example, the kit can contain reagents, tools, and instructions for determining an appropriate therapy for an IBD patient. Such a kit can include reagents for collecting a sample from a patient, such as a swab, and reagents for processing the sample. The kit can also include one or more reagents for detecting the concentration levels of the biomarker. In an alternative embodiment, the kit may also comprise one or more reagents for performing a gene expression analysis, such as reagents for performing RT-PCR, Northern blot, Western blot analysis, or immunohistochemistry to determine biomarker expression levels in a patient sample. Appropriate buffers for the assays can also be included.

A kit can contain separate containers, dividers or compartments for the reagents and informational material.

In particular embodiments, kits for practicing the methods of the invention are provided. Such kits are compatible with both manual and automated immunohistochemistry techniques (e.g., cell staining). These kits comprise at least one antibody capable of specifically binding to at least one of the IBD-specific biomarkers of the present invention. Chemicals for the detection of antibody binding to the biomarker in question, a counterstain, and a bluing agent to facilitate identification of positive staining cells are optionally provided. Alternatively, the immunochemistry kits of the present invention are used in conjunction with commercial antibody binding detection systems, such as, for example the BIOPLEX assay by Bio-Rad. Any chemicals that detect antigen-antibody binding may be used. In some examples, the detection chemicals comprise a labelled polymer conjugated to a secondary antibody. For example, a secondary antibody that is conjugated to an enzyme that catalyses the deposition of a chromogen at the antigen-antibody binding site may be provided. Such enzymes and techniques for using them in the detection of antibody binding are well known in the art. In one example, the kit comprises a secondary antibody that is conjugated to an HRP-labelled polymer. Chromogens compatible with the conjugated enzyme (e.g., DAB in the case of an HRP-labeled secondary antibody) and solutions, such as hydrogen peroxide, for blocking non-specific staining may be further provided. The kits described herein may also comprise a counterstain, such as, for example, hematoxylin. A bluing agent (e.g., ammonium hydroxide) may be further provided in the kit to facilitate detection of positive staining cells.

In another aspect the immunohistochemistry kits of the invention comprise at least two reagents, e.g., antibodies, for the detection of more than one IBD-specific biomarker. Each antibody may be provided in the kit as an individual reagent or, alternatively, as an antibody cocktail comprising all of the antibodies directed to the different biomarkers of interest. Furthermore, any or all of the kit reagents may be provided within containers that protect them from the external environment, such as in sealed containers. Positive and/or negative controls may be included in the kits to validate the activity and correct usage of reagents employed in accordance with the invention. Controls may include samples, such as tissue sections, cells fixed on glass slides, etc., known to be either positive or negative for the biomarker in question. The design and use of controls is standard and well within the routine capabilities of those of ordinary skill in the art.

### EXPERIMENTAL

The present invention describes a new diagnostic method for IBD detection and intestinal inflammation intensity assessment comprising the following key elements that can be combined as a sequence of steps:
1. Non-invasively obtaining by self-collection a sample of material rich in fragments of colonic mucocellular layer by swabbing the anal area of a human subject suspected of being affected with inflammatory bowel disease immediately following bowel opening;
2. Preserving the obtained sample and preparing it for quantitative protein analysis by buffer change followed by the lysis of collected cells, cryopreservation and pre-analysis dilution;
3. Assessing the obtained samples cytologically, wherein cytological patterns can be indicative of the presence or absence of said inflammatory bowel disorder and IBD type (ulcerative colitis or Crohn's disease);
4. Quantitatively determining the levels of biomarkers in said sample, wherein biomarker levels indicate the presence or absence of said inflammatory bowel disorder and provide an estimate of inflammation intensity;
5. Quantitatively determining the levels of biomarkers in said sample, wherein biomarker levels indicate the presence or absence of said inflammatory bowel disorder and provide an estimate of inflammation intensity when the said disorder is ulcerative colitis (UC);
6. Quantitatively determining the levels of biomarkers in said sample, wherein biomarker levels indicate the presence or absence of said inflammatory bowel disorder and provide an estimate of inflammation intensity when the said disorder is Crohn's disease(CD);
7. Determining the levels of biomarkers from the group comprising EDN, calprotectin, protein S100A12, intercellular adhesion molecule 1 (ICAM-1), soluble cytokeratin 18 (epithelial cell death marker), D-dimer (blood coagulation marker), M2-PK, TNFα, pANCA, ASCA, anti-GP2 antibodies, lactoferrin and total human DNA in said samples in order to further characterise IBD type (UC or CD), disease severity, efficiency of applied therapy and probability of response to particular therapeutic schemes;
8. Applying combined detection of levels of a combination or panel of protein biomarkers in said samples for complete characterisation of an individual with diagnosed or suspected IBD in order to determine the precise diagnosis and work out the most suitable therapeutic strategy.

This invention presents a combination of elements that provide a new method for IBD diagnosis, differentiation between IBD types (UC and CD), intestinal inflammation intensity monitoring and treatment efficiency assessment. Application of different biomarkers also provides means for assessing IBD-related damage to colorectal epithelium and selecting adequate therapeutic modalities.

The first key element of the new method is the use of a simple swab designed for non-invasively collecting (self-collecting) material from the anal area of a human subject suspected to have IBD following natural act of defaecation. The principle of the material collection method was described generally in our previous patent application WO2012/150453. The current working version of the swab is depicted in Figure 2. One specific feature of the swab is its spherical shape providing additional uniformity of the area of contact with human body surface during sample collection. The shape and size (8mm in diameter) of the collecting element of the swab also prevent from inadvertently introducing the swab in the anal canal during sample collection. The collecting element of the swab is covered with fibre with hydrophilic properties (flocked nylon) as described by Triva in U.S. Pat. No. 8,317,728. The fibre provides efficient sample collection and easy release of the collected material once the swab is placed in a buffer medium providing cell lysis. The rod of the swab has a breaking point at 100mm from the end of the collecting element (Figure 2). This allows breaking the proximal end of the swab rod off once a collected sample is placed into a 15ml tube containing 3 ml of sample-preserving medium. The swabs prepared for sample collection are sterilised and provided to users sealed in plastic pockets.

The other elements of the invention are further described by referring to examples from a clinical trial that comprised groups of 29 IBD patients, 11 IBS patients and 8 healthy volunteers. The IBD patients all had a confirmed diagnosis of either ulcerative colitis (UC - in 15 cases) or Crohn's disease (CD - in 14 cases) and had presented at the clinic because of a flare-up of their disease. IBS patients were people with colorectal symptoms who were examined by colonoscopy and diagnosed to have IBS (IBD was excluded by clinicians). These people had presented at the clinic due to their symptoms. Control samples were obtained from a group of healthy volunteers without gastrointestinal symptoms or history of any disorders affecting gastrointestinal tract. All samples were taken by patients and healthy controls at home (self-collection). In the IBD group the first sample was taken from each patient prior to them starting on a course of medication intended to control their symptoms. Samples were then taken at day 10, 20, 30 and 60. The aim was to create an initial reference point before treatment (day 0-1) and then sample each patient twice while the symptoms of the flare-up were declining (day 10 and day 20), at day 30, when the patient's symptoms were often resolved and at day 60 in order to provide a follow-up point. Patients with IBS and healthy volunteers (controls) provided samples only once.

Each trial participant was given material collection kits (one kit per collection point) comprising the following essential elements:
- two swabs;
- a sample tube containing material-preserving buffer (see below);
- one sampling card containing two microscope slides with two windows for preparing smears for cytological analysis;
- fixative (spray-bottle) for cytological samples.

All trial participants were instructed to use the kit as follows:
1. Following a bowel movement (before using toilet paper/wipes) to unwrap one swab and gently press the swab tip against the surface of the exterior anal region to collect a sample onto tip (collecting element).
2. To open a preserving medium-containing tube (15ml polypropylene laboratory tube), place the swab into the liquid and break off at the break point. To screw cap tight.
3. To unwrap the second swab and repeat the collection procedure described in (1).
4. To transfer a thin layer of material from the swab tip to both of the sampling card card windows.
5. To spray the windows of the sample card using the fixative-containing sprayer provided.
Preservation of samples at room temperature was achieved by using a preserving medium causing protein stabilisation by precipitation. The medium had the following composition: Tris - 10mM; NaCl - 150mM; EDTA - 10mM; Ammonium Sulfate - saturated; pH - 7.5. 3ml of the preserving medium was used for one collected sample. This sample preservation principle is described in our previous patent application (WO2012/150453).

Following the described collection procedure the samples were sent to the laboratory for analysis. No diagnostic information was provided to laboratory staff before sample analysis was completed and its results recorded.

Upon arrival to the laboratory sample tubes were opened, swabs temporarily removed and carefully placed in empty Eppendorf tubes. The sample tubes were centrifuged at 1920G (4500RPM) for 5 minutes. Following centrifugation the resulting supernatant was carefully removed and discarded. The swabs were returned to the corresponding tubes and 3ml of a lysis buffer were added to each sample tube. Lysis buffer composition was as follows: Tris - 10mM; NaCl - 150mM; Triton X-100 - 0.1%; pH - 7.5M. Accelerated sample lysis was achieved by vigorously shaking the tubes for 15 minutes at 8000rpm using Vortex (Vortex-Genie-2, Scientific Industries Inc.) equipped with a tube-holding rack. Alternatively sample lysis can be achieved by vertical rotation of the tubes for 30 minutes. 200µl aliquots were prepared from each sample. The prepared aliquots were immediately frozen and kept at -80°C until use.

Quantitative protein analysis in sample lysates was performed using commercially available ELISA kits for the following proteins: EDN - EDN ELISA Kit (Medical & Biological Laboratories Co., LTD, Japan); Calprotectin - CALPRO Calprotectin ELISA Test (ALP) (Nova Tec Immunodiagnostica GmbH, Germany); Protein S100A12 - CircuLex S100A12/EN-RAGE ELISA Kit (CycLex Co., Ltd, Japan); ICAM-1 - sE90548Hu Enzyme-linked Immunosorbent Assay Kit for ICAM1 (Uscn Life Science Inc., China/USA); Soluble CK18 - M65 EpiDeath ELISA (PEVIVA AB, Sweden); D-dimer - E90506Hu Enzyme-linked Immunosorbent Assay Kit for D-dimer (Uscn Life Science Inc., China/USA); Glyceraldehyde-3-phosphate dehydrogenase (GAPDH) - GAPDH InstantOne ELISA (eBioscience, Inc., USA). In addition, total protein amount in all samples was determined using QuantiPro BCA Assay Kit (Sigma-Aldrich Co. LLC., USA).

ELISA assays were performed according to manufacturers' instructions. However, sample preparation and dilution had to be optimised for each particular assay. Upon defreezing samples were diluted: 1/5 for EDN and ICAM-1 detection; 1/50 for calprotectin and S100A12 detection, 1/10 for D-dimer and total protein detection. Undiluted samples were used for soluble CK18 and GAPDH detection. All results were expressed as protein concentrations (activity units for CK-18) and recalculated for the original sample lysates presuming that 3ml of the initial preserving medium was replaced with 3ml of the lysis buffer as described above.

Sample preservation and preparation for quantitative biomarker analysis, in particular protein analysis by ELISA comprising steps of buffer change & cell lysis, cryopreservation and pre-analysis dilution constitutes the second key element of the new method.

One of two slides with fixed smears prepared during sample collection was stained with haematoxylin and eosin. The remaining fixed smears were kept at -20°C and used for immunocytochemical visualisation of target proteins when needed. All stained slides were thoroughly analysed microscopically. Photomicrographs have been made using Olympus DP-72 camera.

Figure 3 shows photomicrographs demonstrating cytological patterns in the samples obtained from a healthy volunteer (Figure 3a), a patient with UC (Figure 3b) and a patient with proximal (ileal) CD (Figure 3c). It is evident that colorectal mucocellular layer samples from healthy volunteers contain only small amounts of exfoliated normal colonocytes. In contrast, samples obtained from UC patients (Figure 3b) are characterised by abundant presence of free inflammatory cells, especially polymorhonuclear leukocytes comprising neutrophils (multiple cells with segmented nuclei) and eosinophils. Macrophages, lymphocytes and sometimes mast cells and basophils can also be found. In patients with CD, especially in ileal CD cases partially damaged leukocytes ("leukocyte shadows" shown in Figure 3c) were often observed. It can be concluded that non-invasively collected samples of colorectal mucocellular layer present a good material for diagnostic cytological analysis in the area of IBD.

Key elements 4-7 of the present invention are illustrated by findings of our clinical study presented below.

Results of quantitative assessment of a group of protein biomarkers in healthy volunteers and IBD patients before treatment initiation are presented in Figures 4-11.

The first clinically important endpoint addressed by the present invention is the detection of the presence of an inflammatory process in the gut allowing making a diagnostic conclusion on the presence of IBD. Comparison of concentrations of protein biomarkers in colorectal mucocellular layer samples non-invasively collected from IBD patients (with either CD or UC) or individuals without IBD (control and IBS groups) allowed concluding that both EDN and calprotectin assays demonstrated very good diagnostic performance (see Figures 4-7). EDN assay appeared to be the most efficient among all tested methods. The results of this test generally followed the normal (Gaussian) distribution, and it was remarkable that EDN concentrations were uniformly low in all samples obtained from inflammation-free controls and IBS patients (see Figure 4a). Therefore it was justified to pool results from these two groups together and compare the pooled set to patients with CD, UC and all IBD cases. All EDN concentration results obtained among controls and IBS patients were below 24 ng/ml. This EDN concentration value is slightly above the 0.975 fractile of the distribution of control values often used as the discrimination threshold (cut-off point) in diagnostic tests (Linnet, 1985). Given that numbers of subjects in the study groups were limited, it can be conservatively inferred that the threshold concentration value (cut-off point) for this EDN test should be within the range between 15 ng/ml and 35 ng/ml if the procedure of sample collection, preparation and analysis described in this invention is used. EDN concentration determination used as a diagnostic test was especially efficient in detecting UC (Figure 4a) with perfect (100%) sensitivity and specificity (Figure 4c). This test also performed well for diagnosing CD in most cases (Figure 4a),. When applied for overall IBD detection (see Figures 5a & 5b), EDN measurement showed that average concentration of this protein among IBD patients was 24.9 times higher than among IBD-free individuals (Figure 5a). At the optimal cut-off point of 24ng/ml the EDN-based test provided sensitivity of 86.2% and specificity of 100% for IBD detection (see ROC curve presented in Figure 5b).

The results of calprotectin test also followed the normal (Gaussian) distribution, and calprotectin concentrations were uniformly low (below 4.7 mg/ml) in all in all samples obtained from inflammation-free controls and IBS patients (see Figure 6a). This allowed using the concentration of 4.7µg/ml as the optimal cut-off point worked out in a way similar to that described above for EDN. It could be conservatively inferred that the threshold concentration value (cut-off point) for the calprotectin test should be within the range between 3.5 µg/ml and 6.0 µg/ml if the procedure of sample collection, preparation and analysis described in this invention is used. Calprotectin quantification performance appeared to be slightly inferior compared to EDN for diagnosing UC (Figures 6a & 6c), but detected CD as efficiently as the EDN-based test (compare Figures 6b and 4b). Moreover, calprotectin assay appeared to work better for detecting cases of proximal (ileal) CD (see Figure 6a and corresponding legend). Calprotectin test could also be successfully applied for overall IBD detection (Figures 7a & 7b). Average calprotectin concentration among IBD patients was 9.1 times higher than among IBD-free subjects (Figure 7a). At the optimal cut-off point of 4.7 µg/ml this test had sensitivity of 79.3% and 100% specificity when applied for overall IBD detection (see ROC curve presented in Figure 7b).

Other tested protein biomarkers appeared to be less efficient for diagnosing IBD. Although quantification of both S100A12 (Figure 8) and ICAM1 (Figure 9) allowed to identify most of UC cases, the performance of these tests for CD detection was poor. Moreover, result distributions for these biomarkers deviated from the normal (Gaussian) distribution making the determination of the cut-off points complicated. It should, however, be noted that an obvious result distribution bimodality for these markers existed within the CD group. This bimodality was especially pronounced when ICAM1 assay was applied. No detectable ICAM1 could be found in seven (50%) out of 14 CD cases. In contrast, ICAM1 was detectable and usually present at high concentrations in all patients with UC (Figure 9). The absence of ICAM1 in a significant proportion of CD patients could be used for distinguishing between UC and CD (see below).

The remaining biomarkers (CK-18 and D-dimer) could not be used for IBD diagnosis since their presence was detected only in some (more severe) cases and appeared to be more suitable for IBD treatment efficiency assessment (see below).

The tests based on EDN and calprotectin quantification allowed identifying most IBD cases, but their performance for CD detection, although good, was limited compared to the excellent UC detection rate. It was, however, noticed that different CD cases were missed by the two tests (see legends to Figures 4b and 6b), therefore it appeared to be beneficial to combine the two tests in order to achieve a better rate of CD detection. The outcome of each of the tests could be presented as a ratio of the obtained result and test optimal cut-off point providing the best combination of sensitivity and specificity (in our case - 24ng/ml for EDN and 4.7 µg/ml for calprotectin). In this setting the ratio = 1.0 served as an obvious cut-off point of choice. Once these calculations were done, a pair of ratios became available for each case (Ratio EDN & Ratio Calprotectin). If the higher ratio result was selected as the outcome of this combined test (i.e. Combined Test Result = Ratio EDN if Ratio EDN ≥ Ratio Calprotectin and Combined Test Result = Ratio Calprotectin if Ratio EDN < Ratio Calprotectin), the distributions showed in Figure 10a were obtained. Only one IBD patient (CD case) out of 29 could not be identified using the combined test, which demonstrated performance improvement for both CD detection (Figure 10b shows sensitivity of 92.9% and specificity of 100% for CD) and IBD detection (Figure 10d) shows sensitivity of 96.6% and specificity of 100% for IBD). 100% sensitivity and specificity were obtained when the combined test was applied for UC detection (Figure 10c).

The outcome of the combined test (see Figure 10a) also allowed concluding that test results among patients with UC tended to be higher than in CD cases. The difference between the two subgroups of IBD patients became greater when results of ICAM1 measurement were taken into account. Figure 11a shows a modification of combined test results when an additional multiplier=0.1 was introduced in all cases when no detectable ICAM1 was found in the samples (i.e. ICAM1-Corrected Combined Test Result = Combined Test Result if ICAM1 concentration >0 and ICAM1-Corrected Combined Test Result = Combined Test Result x 0.1 if ICAM1 concentration = 0). ROC curve analysis assessing sensitivity and specificity of the ICAM1-corrected combined test for discriminating UC from CD is shown in Figure 11b The test allowed to achieve 66.7% sensitivity and 100% specificity at cut-off point = 9.0 (area under the curve = 0.919).

Application of protein biomarker measurements in samples of colorectal mucocellular layer obtained using our procedure also allowed assessing inflammation intensity and the extent of colonic epithelium damage/healing during IBD therapy. This approach is exemplified by Figures 12 & 13.

Figure 12a demonstrates EDN concentration measurements made at different time points following treatment initiation in four IBD patients responding well to applied therapy. In all these cases EDN concentrations visibly decreased by day 30 or 60. EDN change patterns in other four patients who did not respond to applied therapeutic interventions are shown in Figure 12b. In these cases EDN levels remain high or even grow indicating the necessity of correcting therapeutic schemes. In particular, late increase of EDN concentration in patient #5 is likely to be an early manifestation of a new relapse (flare-up) of the disease. Patterns obtained in these patients for other inflammation markers (calprotectin, S100A12, ICAM1) were generally similar.

The same cases are presented in Figures 13a and 13b, but for soluble cytokeratin 18 assay, which reflects the degree of colonic mucosa damage (the test specifically detects epithelial cell death). Patterns of changes in CK-18 concentrations generally correlate with EDN changes demonstrating parallelism between colonic inflammation intensity and associated mucosal damage.

Additional analyses of D-dimer concentrations in the collected samples (not shown) suggested that this marker may be useful for detecting the presence of chronic internal bleeding in some cases. Further exploration of this biomarker can help individualising and refining relevant therapeutic schemes.

The use of biomarkers detectable in colonic mucocellular layer samples for therapy efficiency assessment and patient monitoring purposes requires repeated material collections from the same patient providing material for analysis at different time points. Given that the variability of biomarker concentrations among subjects affected with IBD is very high, it appears that comparison of results obtained at different time points and presented as a sequential dynamic curve against therapeutic/clinical parameters should preferably be applied on the individual basis. It might, however, be possible to work out a system of thresholds (cut-off points) for different IBD types (e.g. UC and CD), different inflammation localisations (proximal, distal, pancolitis) and different degrees of IBD severity.

Total protein amount and GAPDH concentration were also detected in the mucocellular layer samples from these patients and controls in order to assess the necessity of introducing a reference assay for the quantitative methods presented above. No correlation between results of these assays and subjects' diagnoses could be found. Total protein assay results were not informative, being influenced by variability of faecal contamination present in some samples. GAPDH concentrations were very low in most samples from all groups and could not be employed as a useful reference standard. At the same time results obtained for EDN, calprotectin, S100A12, ICAM1 and CK-18 (see Figures 4-13) provided good diagnostic performance without introducing additional reference standards.

Presented evidence allows concluding that the invention described in this document provides a new family of methods for inflammatory bowel disease diagnosis and colon inflammation intensity evaluation. The invented methods are based upon quantification of a range of biomarkers in non-invasively collected samples of colonic mucocellular layer. Diagnostic applications of the invented methods comprise: IBD detection, IBD differentiation from non-inflammatory conditions with similar clinical manifestations (in particular IBS) and differentiation between different forms of IBD (UC vs CD). All diagnostic applications are based on collecting and analysing a single sample or a set of samples within a short period of time (e.g. a single defaecation or all bowel movements within 24 hours). The diagnostic tests applied to these samples are designed on the basis of comparing obtained quantitative results against chosen concentration (or activity) thresholds (cut-off points) delimiting positive (i.e. likely to be associated with the presence of the disease) results above the threshold and negative (i.e. likely to be associated with the absence of the disease) results below or equal to the threshold. Applications of the invented methods related to colon inflammation intensity evaluation are based upon repeated collection and analysis of samples performed at different time points over an extended period of time (e.g. therapy course or long-term monitoring of IBD patients in remission). The resulting sequence of test outcomes permits: evaluation of changes in the severity of inflammation as well as related mucosal damage and internal bleeding, assessment of the efficiency of applied therapy and early detection of new IBD flare-ups extremely useful for long-term monitoring of IBD patients in remission. In addition, the approach proposed by the invention can be highly efficient in helping to individualise IBD therapy through determining specific subsets of IBD patients requiring different therapeutic strategies. For example, ICAM-1 quantification (by a single test) can help identifying patients who are likely to respond to already existing therapeutic agents targeting ICAM-1 expression such as alicaforsen (Miner et al, 2006; Vainer, 2010). Likewise, detection of high levels of soluble CK18 can indicate extensive epithelial damage in an IBD patient and help appropriately adjusting applied therapeutic procedures.

It can also be concluded that it may be possible to use biomarkers described in this invention as a biomarker panel for a complete characterisation of an individual with suspected IBD in order to determine the precise diagnosis and work out the most preferable therapeutic strategy. This constitutes the eighth key element of the new method.

## Claims

1. A method for diagnosing an inflammatory bowel disease (IBD), the method comprising:
determining the concentration of at least one IBD-specific biomarker, or a fragment or variant thereof, in a sample of the colonic mucocellular layer obtained from a subject, wherein the sample is obtained from the surface of the anal area following defaecation by taking a swab of said area;
comparing said concentration to a threshold value; and
determining that the subject has IBD when the concentration of at least one IBD-specific biomarker is said sample is equal to or greater than the threshold value;
wherein the at least one IBD-specific biomarker is eosinophil-derived neurotoxin (EDN); and
wherein the threshold value is between 15ng/ml and 35 ng/ml when the collected sample is lysed in 3ml of lysis buffer.

2. The method of claim 1, wherein the threshold value is equal to or greater than 24ng/ml when the collected sample is lysed in 3ml of lysis of buffer.

3. The method of claim 1, wherein the sample is taken from the anal area in the vicinity of the exterior opening of the anal canal.

4. The method of any preceding claim, wherein the concentration of the IBD-specific biomarker is determined by contacting the sample with at least one antibody capable of specifically binding the IBD-specific biomarker or a fragment or variant thereof.

5. A method for monitoring the effectiveness of a treatment for IBD, the method comprising determining the concentration of the EDN protein, or a fragment or variant thereof, in a sample of the colonic mucocellular layer obtained from a subject posttreatment, wherein the sample is obtained from the surface of the anal area following defaecation by taking a swab of said area; and determining that the treatment is effective when the concentration of EDN is less than a value between 35-120 ng/ml when the collected sample is lysed in 3ml of lysis buffer.

6. A method for monitoring for disease relapse in an IBD patient in remission, the method comprising determining the concentration of the EDN protein, or a fragment or variant thereof, in a sample of the colonic mucocellular layer obtained from a subject in remission, wherein the sample is obtained from the surface of the anal area following defaecation by taking a swab of said area, preferably at predetermined intervals; and determining a disease relapse when the concentration of EDN is equal to or greater than a value between 35-120 ng/ml when the collected sample is lysed in 3ml of lysis buffer.

7. A method of differentially diagnosing ulcerative colitis from Crohn's disease, the method comprising
determining the concentration of EDN, calprotectin and ICAM1, or a fragment or variant thereof of either protein, in a sample of colonic mucocellular layer obtained from a subject wherein the sample is obtained from the surface of the anal area following defaecation by taking a swab of said area;
comparing the concentration of EDN and calprotectin to a threshold value to obtain a ratio of EDN concentration/EDN threshold and a ratio of calprotectin concentration/calprotectin threshold;
wherein the threshold value for EDN is 24ng/ml when the collected sample is lysed in 3ml of lysis buffer
and wherein the threshold value for calprotectin is 4.7µg/ml when the collected sample is lysed in 3ml of lysis buffer; and
if ICAM1 cannot be detected in the collected sample then multiplying both ratios by 0.1, or if ICAM1 can be detected in the collected sample then multiplying both ratios by 1;
and determining that the subject has ulcerative colitis when either or both ratio is above 9.0.

## Patentansprüche

1. Verfahren zum Diagnostizieren einer entzündlichen Darmerkrankung (IBD), wobei das Verfahren Folgendes beinhaltet:
Bestimmen der Konzentration von mindestens einem IBD-spezifischen Biomarker, oder eines Fragments oder einer Variante davon, in einer von einer Person gewonnenen Probe der Kolonschleimhautzellschicht, wobei die Probe von der Oberfläche des analen Bereichs nach Defäkation anhand eines Abstrichs des Bereichs gewonnen wird;
Vergleichen der Konzentration mit einem Grenzwert; und
Bestimmen, dass die Person IBD hat, wenn die Konzentration von mindestens einem IBD-spezifischen Biomarker in der Probe gleich dem oder größer als der Grenzwert ist;
wobei der mindestens eine IBD-spezifische Biomarker ein von Eosinophilen abgeleitetes Neurotoxin (EDN) ist; und
wobei der Grenzwert zwischen 15 ng/ml und 35 ng/ml beträgt, wenn die gewonnene Probe in 3 ml Lysepuffer lysiert ist.

2. Verfahren nach Anspruch 1, bei welchem der Grenzwert gleich oder größer als 24 ng/ml beträgt, wenn die gewonnene Probe in 3 ml Lysepuffer lysiert ist.

3. Verfahren nach Anspruch 1, bei welchem die Probe vom Analbereich in der Nähe der äußeren Öffnung des Analkanals genommen wird.

4. Verfahren nach einem der vorhergehenden Ansprüche, bei welchem die Konzentration an IBD-spezifischem Biomarker durch Inkontaktbringen der Probe mit mindestens einem Antikörper bestimmt wird, welcher in der Lage ist, sich spezifisch zu dem IBD-spezifischen Biomarker oder einem Fragment oder einer Variante davon zu binden.

5. Verfahren zum Überwachen der Wirksamkeit einer IBD-Behandlung, wobei das Verfahren das Bestimmen der Konzentration des EDN-Proteins oder eines Fragmentes oder einer Variante davon in einer Probe der von einer Person nach der Behandlung gewonnenen Kolonschleimhautzellschicht , wobei die Probe von der Oberfläche des analen Bereichs nach Defäkation anhand eines Abstrichs des Bereichs gewonnen wird; und das Bestimmen, dass die Behandlung wirksam ist, wenn die Konzentration an EDN geringer als ein Wert zwischen 35-120 ng/ml ist, wenn die gewonnene Probe in 3 ml Lysepuffer lysiert ist, umfasst.

6. Verfahren zum Überwachen eines Rückfalls der Erkrankung bei einem an IBD leidenden Patient, der in Remission ist, wobei das Verfahren das Bestimmen der Konzentration des EDN-Proteins oder eines Fragmentes oder einer Variante davon in einer Probe der von einer Person in Remission gewonnenen Kolonschleimhautzellschicht, wobei die Probe von der Oberfläche des analen Bereichs nach Defäkation anhand eines vorzugsweise zu vorbestimmten Intervallen erfolgenden Abstrichs des Bereichs gewonnen wird; und das Bestimmen, dass ein Rückfall der Erkrankung auftritt, wenn die Konzentration an EDN gleich einem oder großer als ein Wert zwischen 35-120 ng/ml ist, wenn die gewonnene Probe in 3 ml Lysepuffer lysiert ist, umfasst.

7. Verfahren zur Differenzialdiagnose zwischen Colitis ulcerosa und Morbus Crohn, wobei das Verfahren Folgendes beinhaltet:
Bestimmen der Konzentration an EDN, Calprotectin und ICAM1, oder eines Fragments oder einer Variante von einem diese Proteine, in einer von einer Person gewonnenen Probe der Kolonschleimhautzellschicht, wobei die Probe von der Oberfläche des analen Bereichs nach Defäkation anhand eines Abstrichs des Bereichs gewonnen wird;
Vergleichen der Konzentration an EDN und Calprotectin mit einem Grenzwert, um ein Verhältnis EDN-Konzentration/EDN-Grenzwert und ein Verhältnis Calprotectin-Konzentration/Calprotectin-Grenzwert zu erhalten;
wobei der Grenzwert von EDN 24 ng/ml beträgt, wenn die gewonnene Probe in 3 ml Lysepuffer lysiert ist;
und wobei der Grenzwert von Calprotectin 4,7 µg/ml beträgt, wenn die gewonnene Probe in 3 ml Lysepuffer lysiert ist; und
wenn ICAM1 in der gewonnenen Probe nicht festgestellt werden kann, dann Multiplizieren beider Verhältnisse mit 0,1, oder, wenn ICAM1 in der gewonnenen Probe fesgestellt werden kann, dann Multiplizieren beide Verhältnisse mit 1;
und Bestimmen, dass die Person Colitis ulcerosa hat, wenn ein oder beide Verhältnisse über 9,0 betragen.

## Revendications

1. Procédé pour diagnostiquer une maladie inflammatoire de l'intestin (IBD), le procédé comprenant :
la détermination de la concentration d'au moins un biomarqueur spécifique de l'IBD, ou d'un fragment ou variant de celui-ci, dans un échantillon de la couche mucocellulaire du côlon qui est obtenu à partir d'un sujet, dans lequel l'échantillon est obtenu à partir de la surface de la zone anale suite à la défécation en prenant un prélèvement de ladite zone ;
la comparaison de ladite concentration avec une valeur de seuil ; et
la détermination du fait que le sujet présente une IBD lorsque la concentration d'au moins un biomarqueur spécifique de l'IBD dans ledit échantillon est égale ou supérieure à la valeur de seuil ;
dans lequel l'au moins un biomarqueur spécifique de l'IBD est la neurotoxine dérivée des éosinophiles (EDN) ; et
dans lequel la valeur de seuil se situe entre 15 ng/ml et 35 ng/ml lorsque l'échantillon collecté est lysé dans 3 ml de tampon de lyse.

2. Procédé selon la revendication 1, dans lequel la valeur de seuil est égale ou supérieure à 24 ng/ml lorsque l'échantillon collecté est lysé dans 3 ml de tampon de lyse.

3. Procédé selon la revendication 1, dans lequel l'échantillon est pris à partir de la zone anale au voisinage de l'ouverture extérieure du canal anal.

4. Procédé selon l'une quelconque des revendications qui précèdent, dans lequel la concentration du biomarqueur spécifique de l'IBD est déterminée en mettant en contact l'échantillon avec au moins un anticorps capable de se lier de façon spécifique au biomarqueur spécifique de l'IBD ou à un fragment ou variant de celui-ci.

5. Procédé pour surveiller l'efficacité d'un traitement de l'IBD, le procédé comprenant la détermination de la concentration de la protéine EDN, ou d'un fragment ou variant de celle-ci, dans un échantillon de la couche mucocellulaire du côlon qui est obtenu à partir d'un sujet post-traitement, dans lequel l'échantillon est obtenu à partir de la surface de la zone anale suite à la défécation en prenant un prélèvement de ladite zone ; et la détermination du fait que le traitement est efficace lorsque la concentration de l'EDN est inférieure à une valeur qui se situe entre 35 et 120 ng/ml lorsque l'échantillon collecté est lysé dans 3 ml de tampon de lyse.

6. Procédé pour surveiller une récidive de maladie chez un patient présentant une IBD en rémission, le procédé comprenant la détermination de la concentration de la protéine EDN, ou d'un fragment ou variant de celle-ci, dans un échantillon de la couche mucocellulaire du côlon qui est obtenu à partir d'un sujet en rémission, dans lequel l'échantillon est obtenu à partir de la surface de la zone anale suite à la défécation en prenant un prélèvement de ladite zone, de préférence à des intervalles prédéterminés ; et la détermination d'une récidive de maladie lorsque la concentration d'EDN est égale ou supérieure à une valeur qui se situe entre 35 et 120 ng/ml lorsque l'échantillon collecté est lysé dans 3 ml de tampon de lyse.

7. Procédé de diagnostic différentiel de la rectocolite ulcéro-hémorragique et de la maladie de Crohn, le procédé comprenant :
la détermination de la concentration d'EDN, de calprotectine et d'ICAM1, ou d'un fragment ou variant de l'une de ces protéines, dans un échantillon de la couche mucocellulaire du côlon qui est obtenu à partir d'un sujet, dans lequel l'échantillon est obtenu à partir de la surface de la zone anale suite à la défécation en prenant un prélèvement de ladite zone ;
la comparaison de la concentration d'EDN et de calprotectine avec une valeur de seuil pour obtenir un rapport concentration d'EDN/seuil d'EDN et un rapport concentration de calprotectine/seuil de calprotectine ;
dans lequel la valeur de seuil pour l'EDN est de 24 ng/ml lorsque l'échantillon collecté est lysé dans 3 ml de tampon de lyse ;
et dans lequel la valeur de seuil pour la calprotectine est de 4,7 µg/ml lorsque l'échantillon collecté est lysé dans 3 ml de tampon de lyse ; et
si l'ICAM1 ne peut pas être détectée dans l'échantillon collecté, alors la multiplication des deux rapports par 0,1, ou si l'ICAM1 peut être détectée dans l'échantillon collecté, alors la multiplication des deux rapports par 1 ; et
la détermination du fait que le sujet présente une rectocolite ulcéro-hémorragique lorsque l'un des rapports ou les deux sont au-delà de 9,0.
